# EUROPEAN PATENT APPLICATION

(11) **EP 4 721 816 A1**
(43) Date of publication of application: **08.04.2026**
(21) Application number: 24383078.3
(22) Date of filing: 04.10.2024
(51) Int. Cl.: A61P 1/00, A61K 38/51, A61P 3/06, A61P 3/10, C12N 9/88

(54) **ENZYMES CAPABLE OF REDUCING SUCCINATE LEVELS AND USES THEREOF**

(71) Applicant: Fundació Institut d'Investigació Sanitària Pere Virgili (IISPV), 43003 Tarragona (ES); Succipro, SL, 08018 Sant Martí Barcelona (ES); Universitat Rovira i Virgili, 43003 Tarragona (ES); Consorcio Centro de Investigación Biomédica en Red, 28029 Madrid (ES)
(72) Inventor: VENDRELL ORTEGA, Joan, 43005 Tarragona (ES); FERNÁNDEZ VELEDO, Sonia, 43005 Tarragona (ES); SECO MORAL, Jesús, 43005 Tarragona (ES); HUBER RUANO, Isabel, 08018 Barcelona (ES); GRAU BOVÉ, Carme, 08018 Barcelona (ES); VAN BEEK, Hugo Laurens, 9747 AN Groningen (NL); SCHOENAUER, David, 6361 HK Nuth (NL); OLIVEIRA, Eduardo, 6361 HK Nuth (NL)
(74) Representative: Hoffmann Eitle

(57) **Abstract**

The present invention generally refers to enzymes selected from the list consisting of cis-aconitate decarboxylase (SD1) of SEQ ID NO 1, ferulic acid decarboxylase 1 (SD5) of SEQ ID NO 2, or any of sequences SEQ ID NO 3 (SD2), SEQ ID NO 4 (SD3), SEQ ID NO 5 (SD4) or SEQ ID NO 6 (SD6) or modified sequences thereof that have the capacity of reducing succinate levels, for use in the treatment of a disease associated with increased levels of succinate, such as type 2 diabetes mellitus, obesity, non-alcoholic fatty liver disease (NAFLD) and inflammatory bowel disease (IBD).

## Description

### FIELD OF THE INVENTION

The present invention relates to the field of enzymes that have the capacity of reducing succinate levels and uses thereof, particularly, in the treatment of a disease associated with increased levels of succinate, such as type 2 diabetes mellitus, obesity, non-alcoholic fatty liver disease (NAFLD) and inflammatory bowel disease (IBD).

### BACKGROUND ART

Succinate is an intermediate metabolite of the tricarboxylic acid cycle (TCA) that helps regulate cellular nutrient metabolism. It fosters the deposition of skeletal muscle protein, regulates muscle fiber remodeling in exercise state, and regulates glucose homeostasis in obese mice. It also diminishes white adipose tissue deposition. Succinate can also be a target for immune monitoring (Wei YH, et al. Succinate metabolism and its regulation of host-microbe interactions. Gut Microbes. 2023 Jan-Dec;15(1):2190300. doi: 10.1080/19490976.2023.2190300).

In the mitochondria, succinate is metabolized into fumarate by succinate dehydrogenase (SDH), which is an enzyme complex consisting of SDHA, SDHB, SDHC and SDHD subunits (Yang M, Soga T, Pollard PJ. Oncometabolites: linking altered metabolism with cancer. Journal of Clinical Investigation. 2013;123:3652-3658). Apart from metabolism, the role of succinate has been investigated in signal transduction, reactive oxygen species production (ROS), hypoxia inducible factor 1 (HIF-1) activation and stabilization, and G protein-couple receptor-91 (GPR91) stimulation and downstream signaling pathway cascades (Zhao T, Mu X, You Q. Succinate: An initiator in tumorigenesis and progression. Oncotarget. 2017 May 10;8(32):53819-53828. doi: 10.18632/oncotarget. 17734).

Apart from its presence in the mitochondria and in the cytosol, succinate is also present in the intestine and in circulation, as well as in urine. Succinate abnormal accumulation has been found in disease with a component of chronic inflammation (Macias-Ceja DC, et al. Succinate receptor mediates intestinal inflammation and fibrosis. Mucosal Immunol., 2019;12:178-187. doi:10.1038/ s41385-018-0087-3; Ooi M, et al. GC/MS-based profiling of amino acids and TCA cycle-related molecules in ulcerative colitis. Inflamm Res. 2011;60:831-840. doi:10.1007/ s00011-011-0340-7), ischemia (Zhang J, et al. Accumulation of succinate in cardiac ischemia primarily occurs via canonical krebs cycle activity. Cell Rep. 2018;23:2617-2628. doi:10.1016/j.cel rep.2018.04.104), various cancer types (Bardella C, Pollard PJ, Tomlinson I. SDH mutations in cancer. Biochimica et Biophysica Acta (BBA) - Bioenergetics. 2011;1807:1432-1443. doi:10.1016/j.bba bio.2011.07.003; Ricketts C, et al. Germline SDHB mutations and familial renal cell carcinoma. J Natl Cancer Inst. 2008;100:1260-1262. doi:10.1093/jnci/djn254), obesity and type 2 diabetes mellitus (Serena C, et al., Elevated circulating levels of succinate in human obesity are linked to specific gut microbiota. ISMEJ.2018;12(7):1642-1657), non-alcoholic fatty liver disease (Marsal-Beltran A, et al. Protective effects of the succinate/SUCNR1 axis on damaged hepatocytes in NAFLD. Metabolism. 2023 Aug;145:155630. doi: 10.1016/j.metabol.2023.155630) or IBD (Macias-Ceja DC, et al. Succinate receptor mediates intestinal inflammation and fibrosis. Mucosal Immunol., 2019;12:178-187. doi:10.1038/ s41385-018-0087-3). Indeed, succinate can increase the risk of disease progression.

The modulation of succinate circulating and/or intestinal levels with a therapeutic aim has been previously investigated. A clinical study involving lifestyle modification in women with obesity found an association with reduced succinate circulating levels and weight loss and a decrease in the ratio of succinate producers:consumers within the gut microbiota (Serena C, et al., Elevated circulating levels of succinate in human obesity are linked to specific gut microbiota. ISMEJ.2018;12(7):1642-1657); WO2019141780). The administration of *Odoribacter laneus* in murine models of obesity and diabetes was related to reduced blood succinate and improved glucose control and reduced inflammation (Huber-Ruano, et al. Orally administered Odoribacter laneus improves glucose control and inflammatory profile in obese mice by depleting circulating succinate. Microbiome 10, 135 (2022). https://doi.org/10.1186/s40168-022-01306-y). Therefore, there is still an unmet need to provide effective succinate-modulating therapies.

### SUMMARY OF THE INVENTION

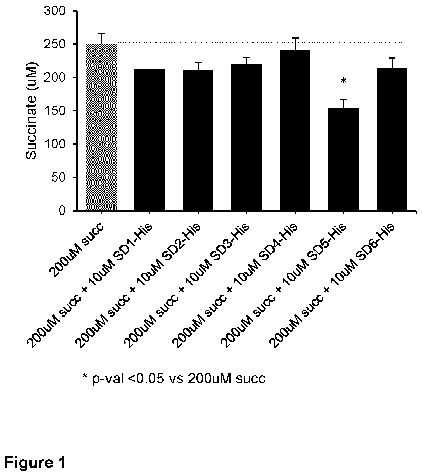

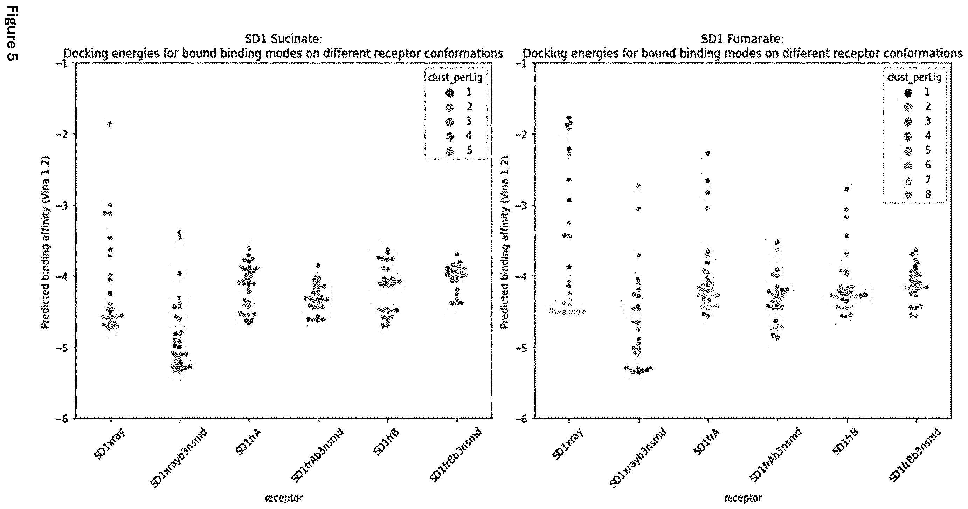

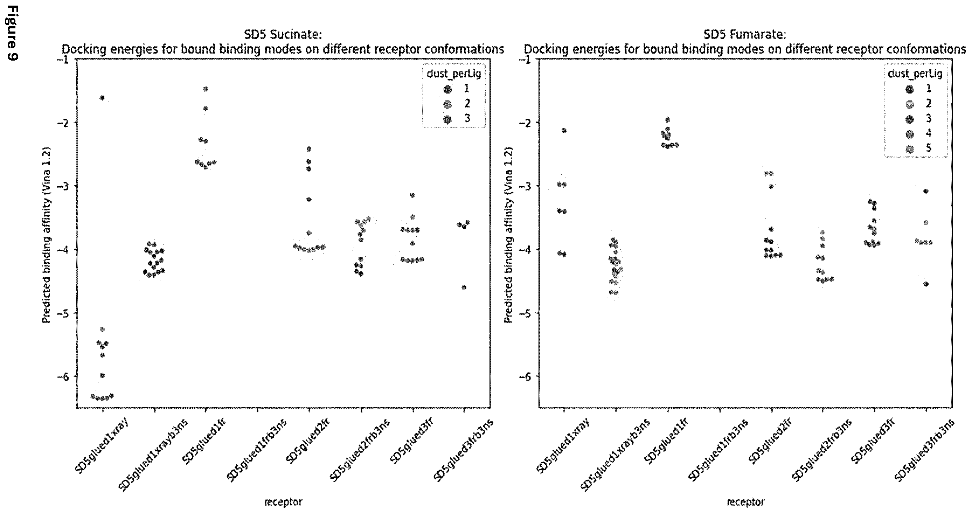

The inventors have identified for the first time that six natural decarboxylases belonging to EC 4.1.1 (carboxy-lyases) subclass, such as cis-aconitate decarboxylase (SD1), gallate decarboxylase (SD2), acetoacetate decarboxylase (SD3), methylmalonyl-coA decarboxylase (SD4), ferulic acid decarboxylase (SD5) and arylmalonate decarboxylase (SD6), showed the capacity of using succinate as a substrate rendering at least one product. Decarboxylases enzymes typically catalyze the removal of a carboxyl group from a substrate but the ability of directly metabolizing succinate as a substate by SD1 to SD6 was not previously described. In particular, SD1 and SD5 have surprisingly shown the capacity of reducing succinate levels *in vitro* (Figs. 1 - 4). The binding affinity of succinate and fumarate was predicted in silico and showed that both metabolites use SD1's active site of its natural substrate (cis-aconitate) to similarly stabilize succinate and fumarate (Fig.5; Fig. 6; Fig. 7 and Fig.8). For SD5, Molecular Dynamics simulations showed similar predicted binding energies for both succinate and fumarate (Fig. 9; Fig. 10; Fig. 11)

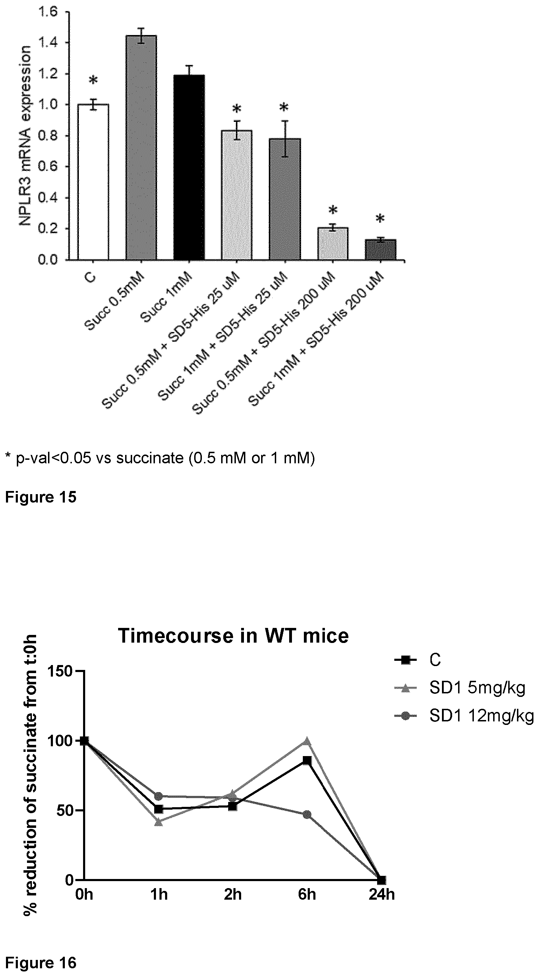

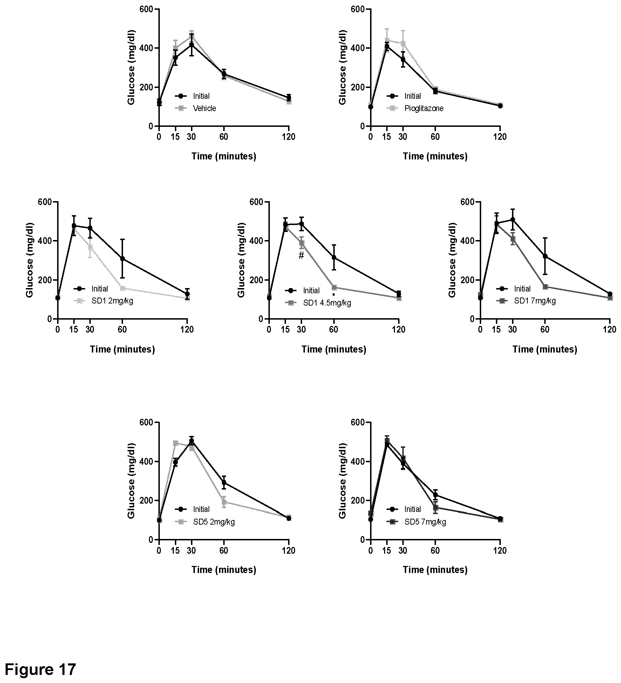

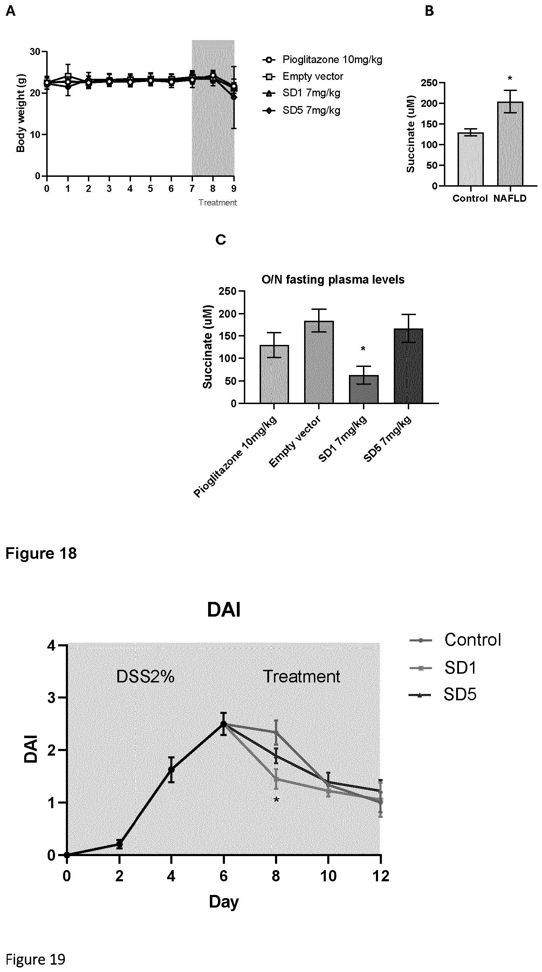

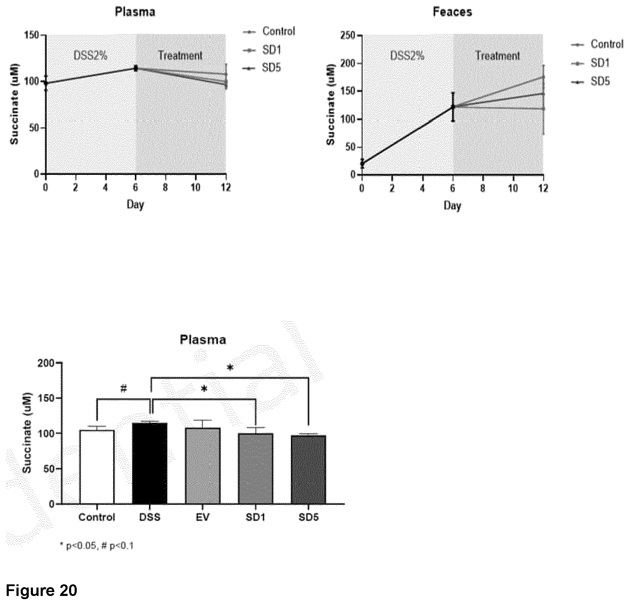

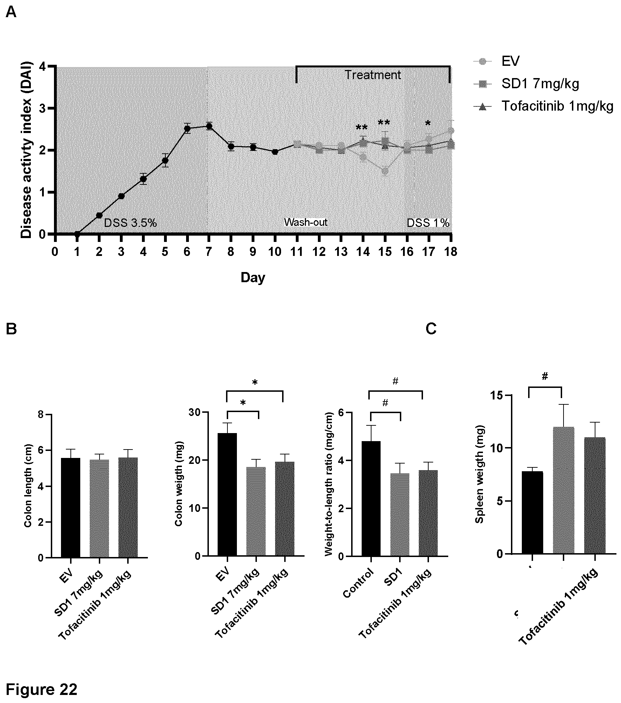

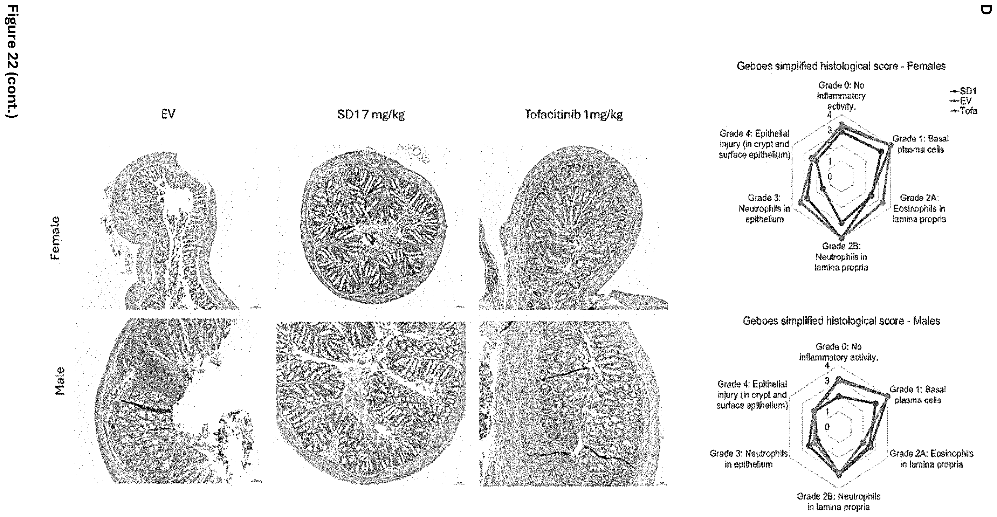

Importantly, SD1 was found to reducesuccinate levels in a murine model of obesity and type 2 diabetes. More specifically, the recovery of succinate levels 6 hours after fasting was reduced (Fig. 16). SD1 and SD5 reduced succinate in a NAFLD model (Fig. 18), whereasSD1 ameliorated glucose tolerance in NAFLD (Fig. 17). SD1 also reduced disease activity in moderate acute colitis (Fig. 19) and severe flare-relapse colitis (Fig. 22). Both SD1 and SD5 reduced plasma and fecal levels of succinate in DSS-induced colitis (Fig. 20). Thus, the inventors have demonstrated the *in vitro* use of SD1 and SD5 and their variants and the *in vivo* use of SD1 and SD5 to reduce the levels of succinate.

### BRIEF DESCRIPTION OF THE FIGURES

**Figure 1****.** SDs cloned and produced in Escherichia coli reduce succinate levels. Different amounts of enzyme were incubated in different conditions for 24 hours. Reactions were stopped by incubating 20min at 80°. The amount of succinate remaining after each reaction was measured with EnzyChromTM Succinate Assay Kit following manufacturer instructions. Data are presented as mean ± Standard Error of the Mean (SEM). A statistically significant decrease in succinate concentration compared to the control (200 µM succinate) is observed with the SD5-His enzyme (*p < 0.05).

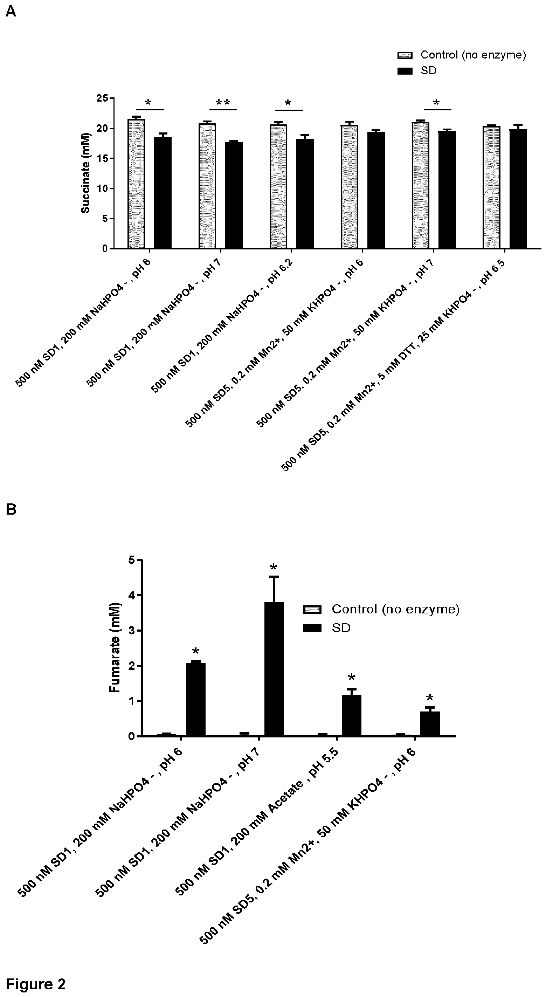

**Figure 2****.** SD1 decreases succinate generating fumarate in 24-hour reactions in different buffer conditions. (A) Succinate concentration (mM) and (B) fumarate concentrations (mM) after 24-hour reactions with 500 nM SD1 under various buffer conditions compared to control (no enzyme). Statistically significant decreases in succinate and increases in fumarate concentration compared to the control are observed in several buffer conditions. Data are presented as mean ± SEM. Statistically significant differences compared to the control are indicated (*p < 0.05, **p < 0.01).

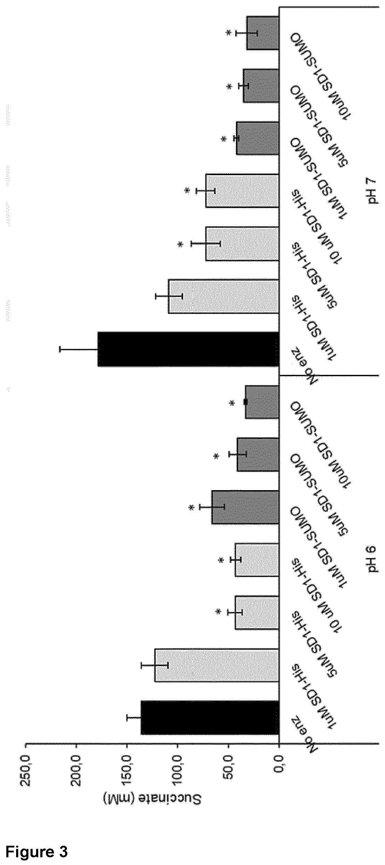

**Figure 3****.** Succinate levels significantly decrease in the presence of SD1-His and SD1-SUMO at pH 6 and pH 7. Succinate concentration (mM) after 24-hour reactions are reduced with varying concentrations of SD1 with His or SUMO tags at pH 6 and pH 7, compared to a control with no enzyme. Significant reductions in succinate levels are observed with 1 µM, 5 µM, and 10 µM of SD1-His and SD1-SUMO at both pH levels (*p < 0.05). Data are presented as mean ± SEM.

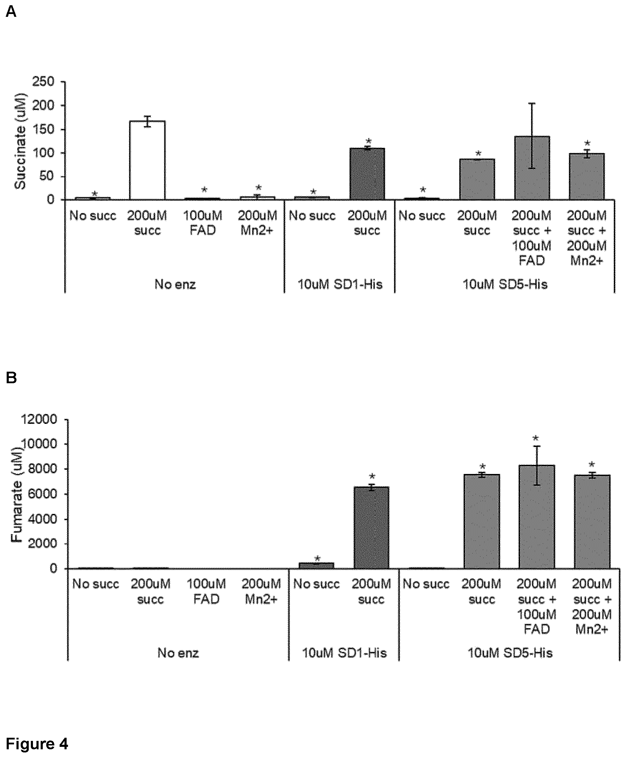

**Figure 4****.** Succinate decrease and fumarate increase by SD1 and SD5 is not altered by the addition of cofactors in the reaction. (A) Succinate (uM) and (B) Fumarate (uM) levels after 24-hour reactions of SDs with 200uM succinate compared to control of 200uM succinate (without enzyme). The addition of FAD and Mn2+ as cofactors did not alter the reaction. Data are presented as mean ± SEM. Statistically significant differences compared to the control (200 uM succinate without enzyme) are indicated (*p < 0.05).
**Figure 5****.** Predicted binding affinity for succinate and fumarate on different conformations of SD1. The binding modes were clustered according to the different geometries (differently colored dots).

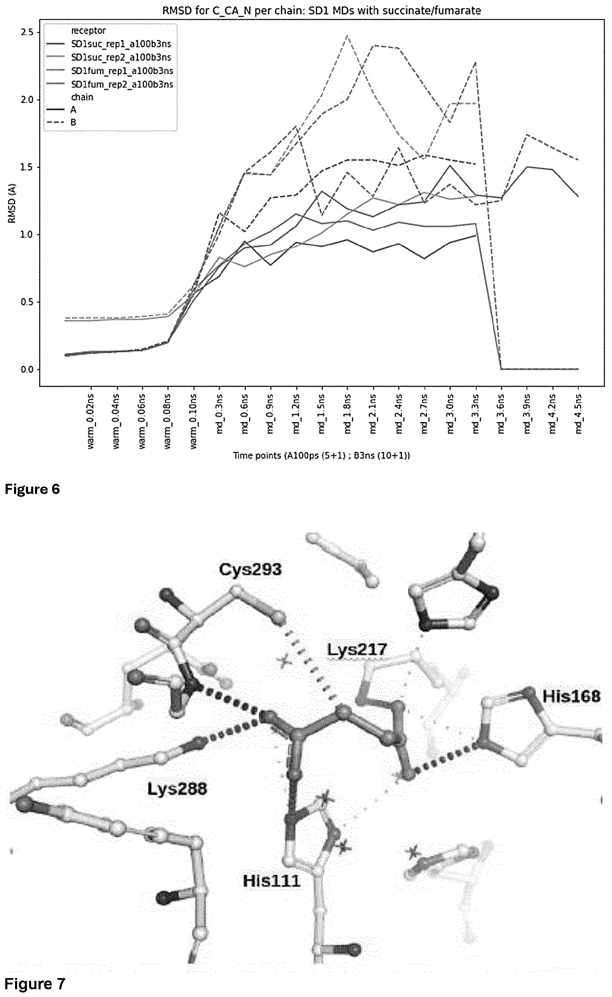

**Figure 6****.** Evolution of the RMSD (root mean square deviation) of the backbone from each SD1 monomer along the several molecular dynamics simulations
**Figure 7****.** Representation of the binding mode of succinate (orange) after equilibration of molecular dynamics simulations of 3 active sites conformations of SD1.

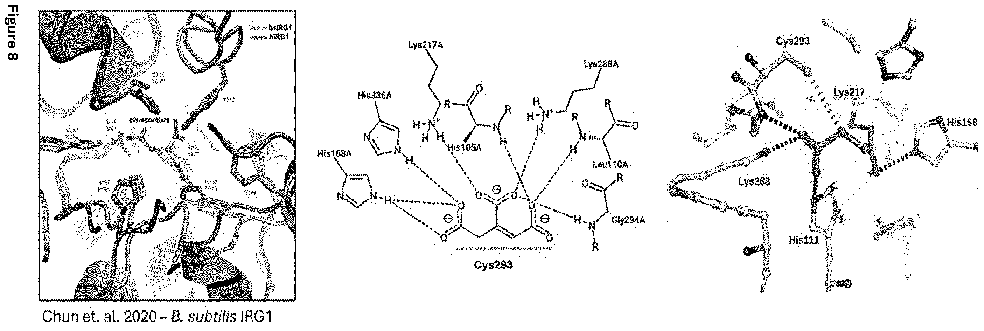

**Figure 8****.** Interactions between cis-aconitate with SD1 active site and representation of the binding mode of succinate after equilibration Molecular Dynamics simulations for 3 ns.
**Figure 9****.** Predicted binding affinity for succinate and fumarate on different conformations of SD5. The binding modes were clustered according to the different geometries (differently colored dots).

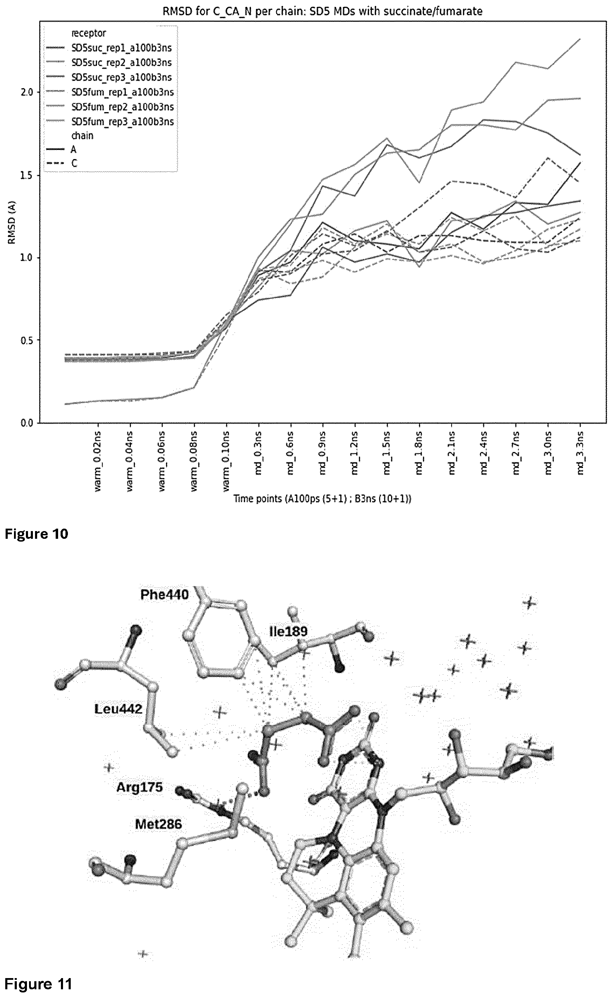

**Figure 10****.** Evolution of the RMSD of the backbone from each monomer along several molecular dynamics simulations of SD5 with succinate or fumarate.
**Figure 11****.** Representation of the interactions between succinate (orange) acid with SD5 active site after equilibration for 3 active sites conformations molecular dynamics simulations.

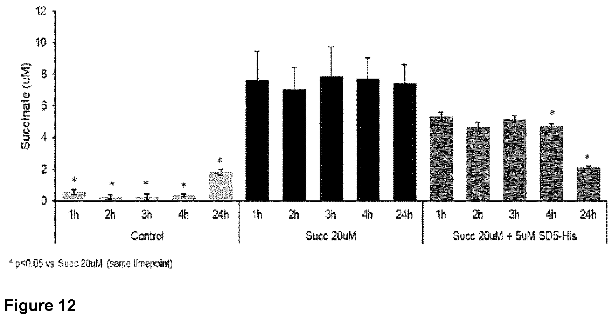

**Figure 12****.** SD5 reduces succinate levels after 24h treatment with succinate in HepG2 cells. HepG2 cells were treated with 20uM succinate and/or 5uM SD5. Succinate levels of succinate + SD5 treated cells were significantly lower after 4h and 24h (* p<0.05).
Data are presented as mean ± SEM.

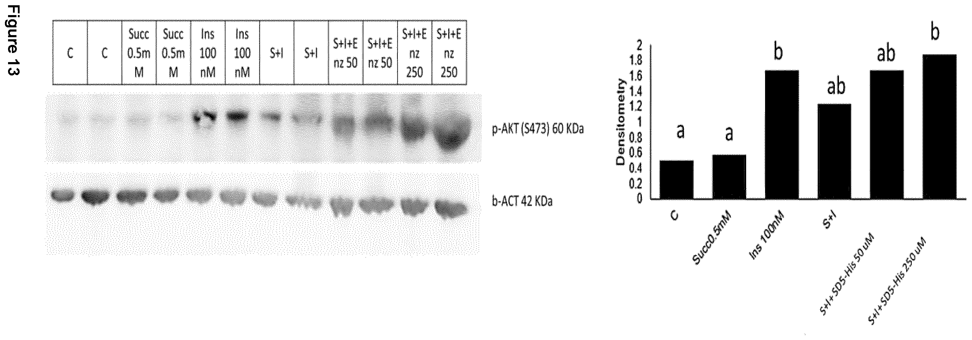

**Figure 13****.** SD5 treatment in HepG2 cells recovers insulin signaling blocked by succinate. HepG2 cells were treated for 24h with insulin and/or Succinate and/or SD5.
Succinate blocks insulin induced phophorylation of AKT as SD5 recovers AKT phosphorylation. Data are presented as mean ± SEM. Statistically different groups by one-way ANOVA are represented with letters (p-val<0.05).

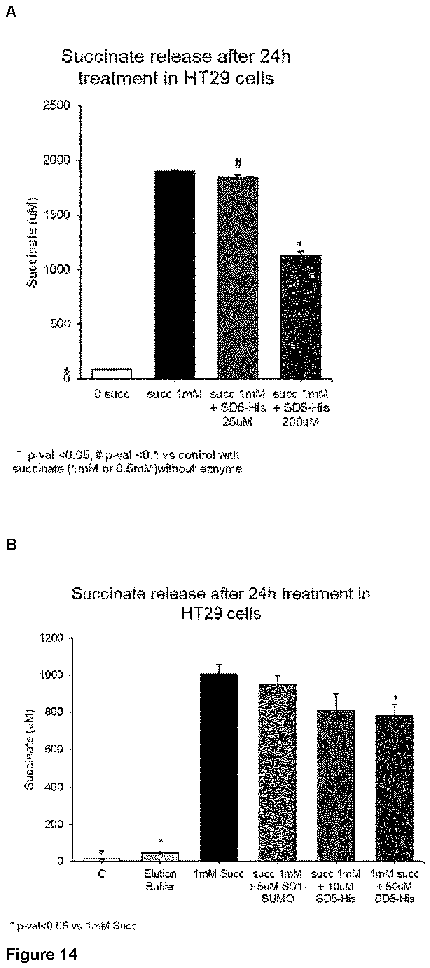

**Figure 14****.** SD1 and SD5 reduce succinate levels after 24h treatment with succinate in HT29 cells. HT29 cells were treated with 1mM succinate and/or different concentrations of SD1 and SD5. Data are presented as mean ± SEM. Statistically significant differences compared to the control (1 mM succinate without enzyme) are indicated (*p < 0.05).
**Figure 15****.** SD5 reduce succinate-induced inflammasome marker NPLR3 expression after 24h treatment in HT29 cells. HT29 cells were treated with 0.5 or 1mM succinate and/or different concentrations of SD5. Data are presented as mean ± SEM. Statistically significant differences compared to the control (0.5 mM or 1 mM succinate without enzyme) are indicated (*p < 0.05).
**Figure 16****.** SD1 injection in diabetic mice prevents succinate increase after at 6h. SD1 was injected at two different doses intraperitoneally to non-fasted db/db mice. Blood extractions at different time points were performed from the saphenous vein and succinate was measured from plasma. At 6h, mice injected with the higher dose of SD1 (12 mg/kg) showed reduced succinate levels compared to the control.
**Figure 17****.** One week of SD1 treatment (4.5 mg/kg) in mice with diet-induced NAFLD reduced their glucose intolerance, opposite to Pioglitazone 10 mg/kg, a drug used for type 2 Diabetes, or the vehicle. Data are presented as mean ± SEM. Statistically significant differences compared to the initial succinate levels (before treatment) are indicated (# p<0.1, *p < 0.05).
**Figure 18****.** SD1 is able to reduce succinate levels that are increased in mice with diet-induced NAFLD. (A) Body weight during 7 weeks of choline-deficient and low methionine (0.1%) high fat (60%) diet and 2 weeks of treatment + diet were not altered. (B) Succinate levels were increased in plasma (Mean + s.e.m *p<0.05 unpaired t-test). (C) Plasma succinate levels after one week of treatment with SD1 (7 mg/kg) are reduced in comparison to the vehicle and to Pioglitazone (10 mg/kg) (Mean + s.e.m *p<0.05 vs vehicle in Tukey's post-hoc test, one-way ANOVA).
**Figure 19****.** SD1 reduces disease activity index (DAI) in mice with DSS-induced acute colitis. Colitis was induced with administration of 2% of Dextran Sodium Sulfate (DSS) to drinking water for 6 days. An intracolonic administration of SD1 was able to reduce DAI in two days.
**Figure 20****.** SD1 is able to reduce succinate levels that are increased in mice with DSS-induced acute colitis. Succinate levels were increased in faeces and plasma after DSS administration. Levels were significantly lower in SD1- and SD5-treated mice. (Mean + s.e.m *p<0.05 unpaired t-test).

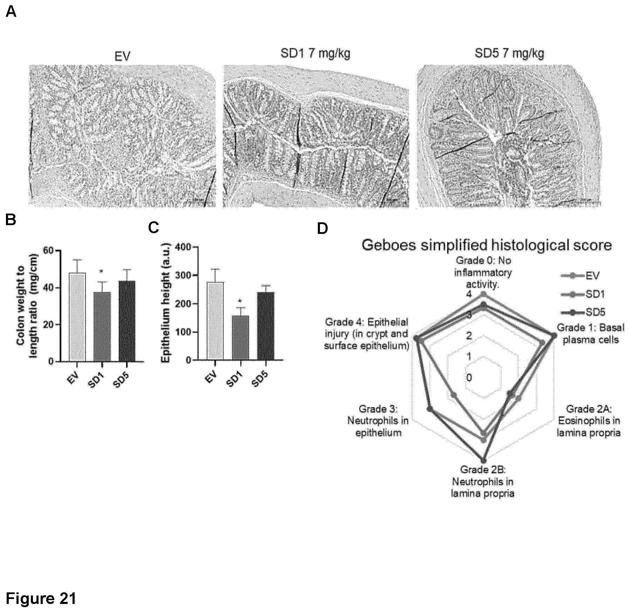

**Figure 21****.** SD ameliorated mucosal healing in mice with DSS-induced acute colitis. (A) H&E staining of colon tissue (x 20). (D) Geboes simplified histological score represented by radar chart reveals lower damage in mice treated with SD1 as seen by lower inflammatory cells infiltration and injuries. (B) Colon weight to length ratio evaluated from histological colon. (C) Epithelium height evaluated from histological colon. All results are expressed as mean ± SEM. *p < 0.05 versus EV mice (unpaired t-test).
**Figure 22****.** SD1 treatment prevents DSS-induced colitis relapse in mice. (A) Disease activity index (DAI) was recorded every day since the induction of colitis with DSS in female and male mice. After a first period of 7 days of induction with DSS 3.5%, DAI was reduced during a wash-out period. Since day 11, intracolonic treatments with vehicle (empty vector, EV), SD1 7mg/kg and a comparator (Tofacitinib 1mg/kg) were administered daily. At day 16, a colitis relapse was induced with DSS 1% for four days. During this period, a preventive effect of SD1 was observed. At the end of the experiment, tissues weight and length were recorded. (B) Colons were shorter and showed and improved length to weight ratio in SD1 and Tofacitinib treated mice. (C). Spleen's length and weight were increased in mice treated with SD1 and Tofacitinib, respectively. (D) H&E staining of colon tissue and Geboes simplified histological score represented by radar chart reveals lower damage in mice treated with SD1.

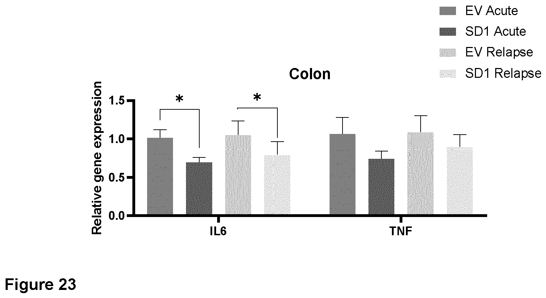

**Figure 23****.** SD1 reduces expression of inflammatory markers IL6 and TNF in both acute and flare-relapse colitis models

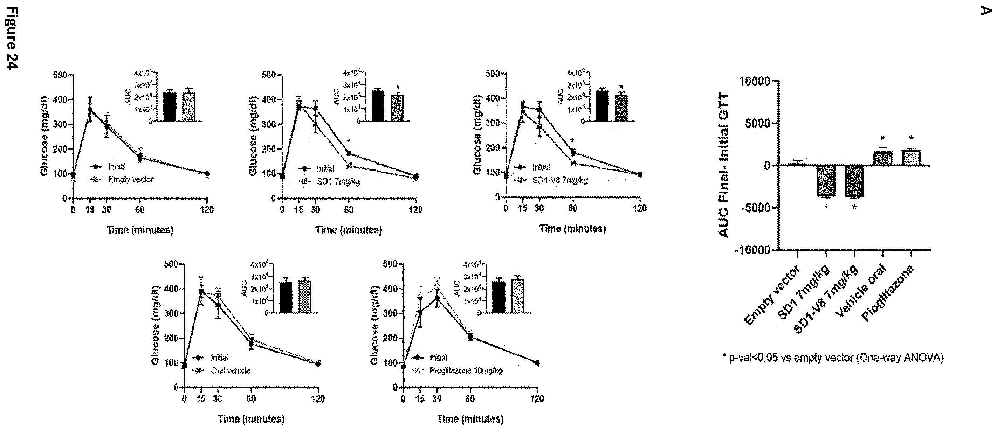

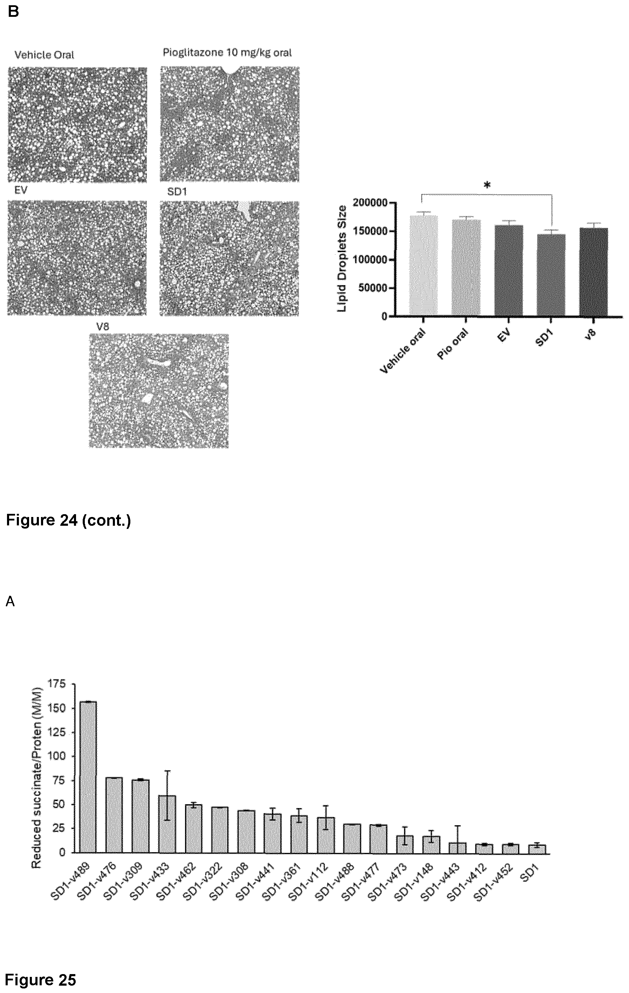

**Figure 24****.** SD1 and SD1 variants intracolonic administration in mice diet-induced NAFLD are able to ameliorate glucose tolerance and steatosis. (A). Glucose tolerance test before and after 2 weeks of treatment reveal improved glucose tolerance with SD1 and SD1v489 (labelled as v8 in the graph) treated mice. (B) H&E staining reveals reduced lipid droplet size in SD1-treated mice.

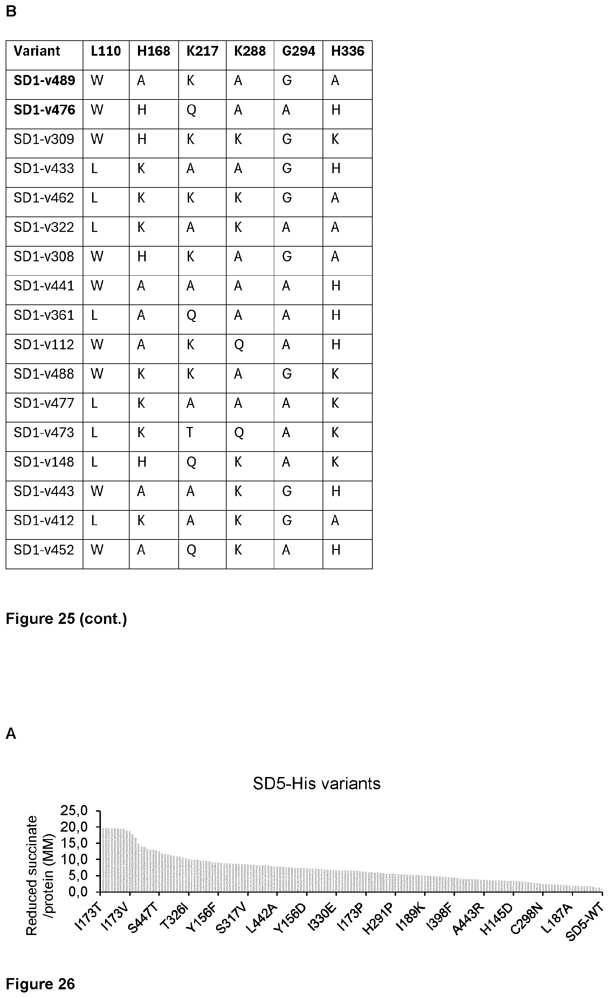

**Figure 25****.** Generation of SD1 variants with improved succinate degradation capacity. (A) Enzymatic in vitro reactions showed that succinate reduction capacity in of wild-type SD1 (blue) is improved in variants generated by semirational mutagenesis. (B) Table of translated mutations in variants from graph A in comparison with the sequence in six positions of wild-type SD1.

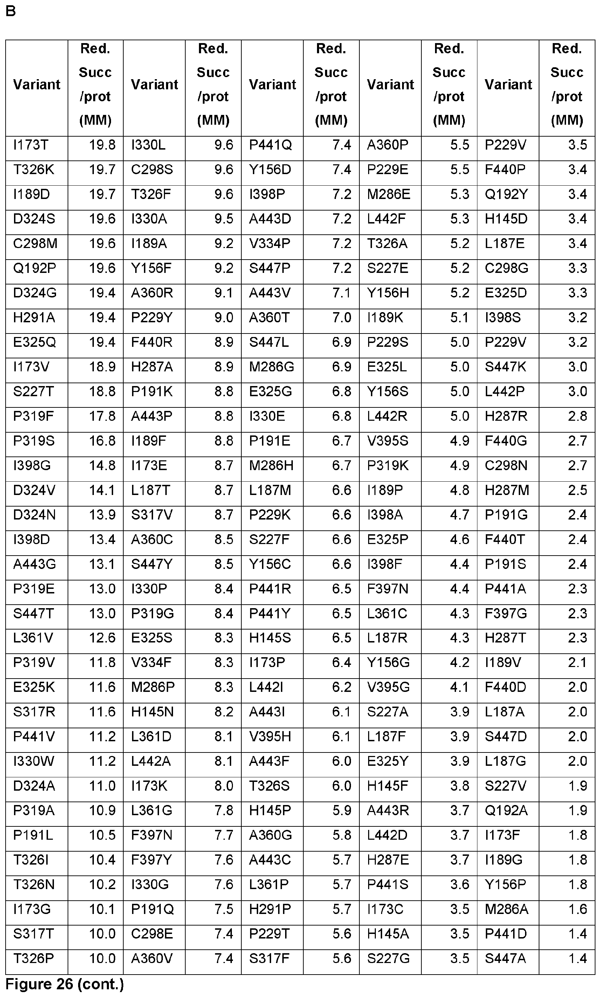

**Figure 26****.** Generation of SD5 variants. Enzymatic in vitro reactions showed that succinate reduction capacity of wild-type SD5 is improved in variants of generated by semirational mutagenesis. A graph (A) and a table (B) of succinate-relative degrading activities is shown.

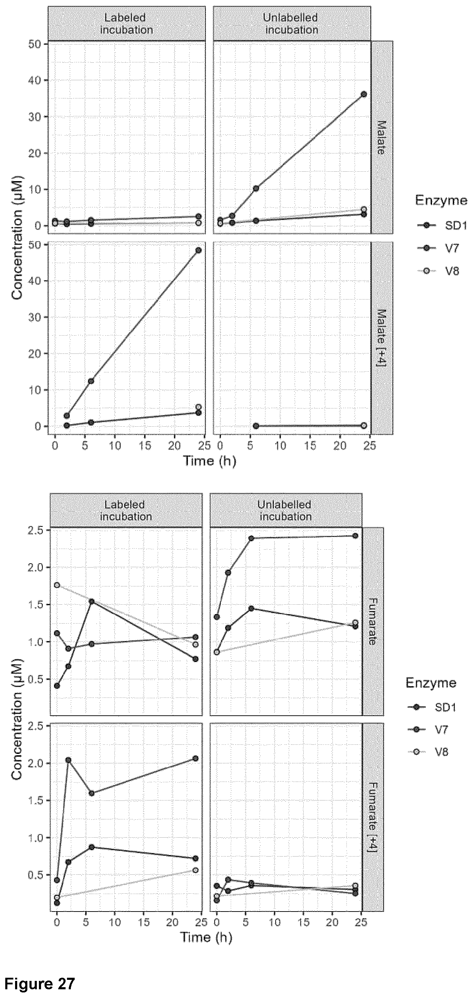

**Figure 27****.** Time-course of the enzymatic reaction of SD1 and two variants (V476 and V489). The enzymes were incubated with succinate or succinate-C13 (labelled succinate). Reactions were stopped at different times. Both fumarate and malate were detected over time as a reaction products albeit at a different rate depending on the enzyme.

### DEFINITIONS

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof' is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context. As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, 10, 11, 12, 13, 14 or 15%. Preferably the term "about" means exactly the indicated value (± 0%).

An "enzyme" is a protein that catalyzes a chemical reaction upon substrate molecules and reduces the activation energy necessary for said chemical reaction to take place by stabilizing the transition state. In a first step, the enzyme binds to a substrate to form an enzyme-substrate [ES] complex reaction. The increase of the concentration of the substrate [S] will increase the speed of the reaction until the maximum velocity is reached. After the ES formation, a product generates that dissociates from the enzyme (Lewis T, Stone WL. Biochemistry, Proteins Enzymes. [Updated 2023 Apr 24]. In: StatPearls [Internet]. Treasure Island (FL): StatPearls Publishing; 2024 Jan-. Available from: https://www.ncbi.nlm.nih.gov/books/NBK554481/.)

The Enzyme Classification (EC) is based on the recommendations of the Nomenclature Committee of the International Union of Biochemistry and Molecular Biology (IUBMB). The Protein Data Bank (PDB) assigns EC (Enzyme Commission) numbers to protein chains in structures according to the type of chemical reaction catalyzed, specific donors and receptors of chemical groups participating in the reactions etc., all based on information from UniProtKB, GenBank, KEGG or are author specifications. The Enzyme Commission (EC) classification groups enzymes that perform the same or related enzymatic functions (Protein Data Bank. Enzyme Classification. Updated 8.12.2021, retrieved 04.06.2024 from https://www.rcsb.org/docs/search-and-browse/browse-options/enzyme-classification). The classification depicts the enzyme names using a series of four numbers. The first number defines the enzyme category for the reaction type: oxidoreductases, transferases, hydrolases, lyases, isomerases, ligases, and translocases (Table 1). The second number denotes information about the compounds or groups, while the third specifies the type of reaction. The fourth number is specific, giving each enzyme a number akin to a serial number (Ann Benore, M. (2019), What is in a name? (or a number?): The updated enzyme classifications. Biochem Mol Biol Educ, 47: 481-483. https://doi.org/10.1002/bmb.21251).

**Table 1. The enzyme classification**

| **Class number** | **Name** |
|---|---|
| 1 | oxidoreductases |
| 2 | transferases |
| 3 | hydrolases |
| 4 | lyases |
| 5 | isomerases |
| 6 | ligases |
| 7 | translocases |

Lyases (EC. 4) are enzymes that cleave C-C, C-O, C-N and other bonds by means other than by hydrolysis or oxidation. They differ from other enzymes in that two (or more) substrates are involved in one reaction direction, but there is one compound fewer in the other direction. When acting on the single substrate, a molecule is eliminated and this generates either a new double bond or a new ring. The systematic name is formed according to 'substrate group-lyase'. In common names, expressions like decarboxylase, aldolase, etc. are used. (ExplorEnz. EC 4. Lyases. Accessed 04.06.2024, retrieved from https://www.enzyme-database.orq/cinfo.php?c=4&sc=0&ssc=).

Carbon-carbon lyases (EC. 4.1) is a subclass that contains the decarboxylases (carboxy-lyases; EC 4.1.1), the aldehyde-lyases, which catalyse the reversal of an aldol condensation (EC 4.1.2), the oxo-acid-lyases, which catalyse the cleavage of a 3-hydroxy acid (EC 4.1.3) and other carbon-carbon lyases (EC 4.1.99), or the reverse reactions (ExplorEnz. EC 4. Lyases. Accessed 04.06.2024, retrieved from https://www.enzyme-database.org/cinfo.php?c=4&sc=1&ssc=).

"Decarboxylation" in the present invention is understood as the removal of carbon dioxide from organic acids.

A "decarboxylase enzyme" is an enzyme capable of or having the capacity of removing carbon dioxide from organic acids.

A "carboxy-lyase" is an enzyme that catalyze the addition of a carboxyl group to a compound (carboxylases) or the removal of a carboxyl group from a compound (decarboxylases). Carboxy-lyases pertain to EC 4.1.1 subclass.

Succinic acid (also named butanedioic acid, C₄H₆O₄) is a symmetric, four-carbon dicarboxylic acid. It forms colorless, odorless crystals with an acid taste.

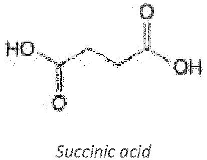

In living organisms it exists as succinate (also named as butanedioate (IUPAC), succinate dianion, butanedioic acid, ion(2-), succinate ion, succinic acid, ion(2-), monosuccinate, 1,2 ethanedicarboxyulic acid, succinate(2-), butanedioate(2-), succinate ion(2-) among other synonyms). Its molecular formula is C₄H₄O₄⁻² and its molecular weight is 116.07 g/mol (PubChem. Succinate. Accessed 05.06.2024, retrieved from https://pubchem.ncbi.nlm.nih.gov/compound/succinate).

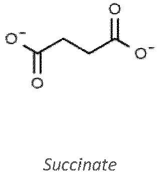

Succinate is an intermediate of the tricarboxylic acid (TCA) cycle. It is generated from α-ketoglutarate (AKG) by 2-oxolgutarate dehydrogenase (OSDGH) and succinyl-CoA synthetase (SCS). Succinate is a substrate of succinate dehydrogenase (SDH), which oxidizes succinate into fumarate. This process leads to the donation of two hydrogens to the respiratory chain, playing a key role in the generation of adenosine triphosphate (ATP) in mitochondria.

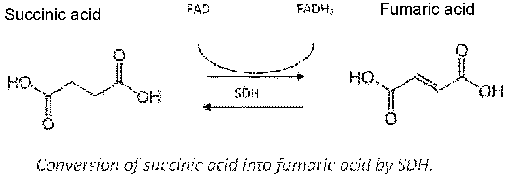

Another source or succinate production is tne syntnesis from glutamine and its use through anaplerosis to produce AKG. Outside the TCA cycle, glutamate is metabolized into succinic semialdehyde by the "γ-aminobutyric acid (GABA) shunt". Succinic semialdehyde is converted to succinate by succinic semialdehyde dehydrogenase and vitamin B₁₂. Succinate is then oxidized to fumarate by succinate dehydrogenase. (Tannahill GM, Curtis AM, Adamik J, Palsson-McDermott EM, McGettrick AF, Goel G, Frezza C, Bernard NJ, Kelly B, Foley NH, et al. Succinate is an inflammatory signal that induces IL-1β through HIF-1α. Nature. 2013;496:238-242. doi: 10.1038/nature11986; Wong CG, Bottiglieri T, Snead OC 3rd. GABA, gamma-hydroxybutyric acid, and neurological disease. Ann Neurol. 2003;54(Suppl 6):S3-12. doi: 10.1002/ana.10696.)

In addition, succinate is an intermediate metabolite in the fermentation of dietary and host-derived carbohydrates by gut microbes. Intestinal microorganisms such as *Bacteroides spp., Prevotella spp., Firmicutes spp.,* and *Veillonella spp.* produce propionate from dietary carbohydrates via the succinate pathway. Indigestible carbohydrates are converted into phosphoenolpyruvate (PEP), subsequently converted into oxaloacetate (OAA) in the presence of carbon dioxide. OAA is converted to succinate through the activation of malate dehydrogenase and fumarate dehydrogenase. Succinate is then converted to succinyl-coA and afterwards to methylmalonate (MAA) with the involvement of vitamin B₁₂. Propionate is produced from MAA conversion (Reichardt N, Duncan SH, Young P, Belenguer A, McWilliam Leitch C, Scott KP, Flint HJ, Louis P. Phylogenetic distribution of three pathways for propionate production within the human gut microbiota. Isme J. 2014;8:1323-1335. doi: 10.1038/ismej.2014.14.; Krautkramer KA, Fan J, Backhed F. Gut microbial metabolites as multi-kingdom intermediates. Nat Rev Microbiol. 2021;19:77-94. doi: 10.1038/s41579-020-0438-4).

Species such as *Propionibacterium granulosum* feed on food in the gut to synthetize and store glycogen during host feeding. During host fasting, said bacteria convert glucose into malate, which can be metabolized into propionate or pyruvate. Pyruvate is converted into acetyl-coA. Acetate:succinate CoA-transferase (ASCT) transfers the CoA moiety from acetyl-coA into succinate thereby producing succinyl-coA and acetate. Succinate can also be generated using acetate as a substrate. The bacteria *Acetobacter aceti* use ASCT to replace the TCA cycle succinyl-coA synthetase (SCS) to produce succinate from succinyl-coA to complete the TCA cycle. (Kwong WK, Zheng H, Moran NA. Convergent evolution of a modified, acetate-driven TCA cycle in bacteria. Nat Microbiol. 2017;2:17067. doi: 10.1038/nmicrobiol.2017.67.)

*Prevotellaceae spp.,* among others, convert succinate into succinyl-coA through the succinate pathway described for propionate producers, but with the difference that succinyl-coA is converted into succinate semialdehyde by succinate semialdehyde dehydrogenase. Butyrate is produced through a series of reactions (Louis P, Flint HJ. Formation of propionate and butyrate by the human colonic microbiota. Environ Microbiol. 2017;19:29-41. doi: 10.1111/1462-2920.13589).

The term *"in vitro",* as used herein, refers to the fact that the method is not carried out directly in a human or animal subject, but preferably, but not limited to, cells, samples or fluids isolated from a subject, preferably a human subject, or in a test tube or in any other medium.

"Circulating succinate", as used in the present invention, refers to the levels of succinate in blood. The source of circulating succinate remains unclear: it can derive from damaged tissues but also from intestinal efflux by specific gut microbiota (Ariza AC, Deen PM, Robben JH. The succinate receptor as a novel therapeutic target for oxidative and metabolic stress-related conditions. Front Endocrinol (Lausanne). 2012;3:22 ; Deen PM, Robben JH. Succinate receptors in the kidney. J Am Soc Nephrol. 2011;22:1416-22; Sharon G, Garg N, Debelius J, Knight R, Dorrestein PC, Mazmanian SK. Specialized metabolites from the microbiome in health and disease. Cell Metab. 2014;20:719-30). Circulating succinate levels were significantly increased in obesity (-50-125 µM) and type 2 diabetes (-75-150 µM) patients compared to lean subjects (-0-50 µM), as measured by a fluorimetric method (EnzyChrom^{™} Succinate Assay Kit, BioAssay Systems, Hayward, CA) and validated by liquid chromatography tandem-mass spectrometry (LC-MS/MS) and nuclear magnetic resonance (NMR) analysis (WO2019/141780; Serena, C. et al. (2018) Elevated circulating levels of succinate in human obesity are linked to specific gut microbiota. ISME J. 12, 1642-1657). In addition, serum succinate levels were found significantly higher in samples from Crohn's disease (-0.02 mM) patients than in serum from healthy donors (-0.01 mM). Succinate levels in intestinal mucosa from Crohn's disease (-1.0 µmol/g) patients were significantly higher than the ones from non-IBD (-0.25 µmol/g) patients, determined by ¹H NMR Spectroscopy (Macias-Ceja et al., 2018).

A "wild-type" sequence is understood in the present invention as the natural sequence, for instance, a natural form of a protein sequence, without genetic mutations.

A "sequence with at least 85% identity" with another sequence is understood throughout the present invention as retaining, maintaining or even improving the functional properties as the wild-type sequence.

In the present invention, the term "amino acid" refers to a molecule containing both an amino group and a carboxyl group. Amino acids can be classified by the side chain group. There are basically four different classes of amino acids determined by different side chains: (1) nonpolar, (2) polar and neutral, (3) acidic and polar, (4) basic and polar.

Suitable amino acids include, without limitation, alpha amino acids, such as the L-isomers of alpha-amino acids of the 20 common naturally occurring alpha-amino acids: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine; natural beta-amino acids (e.g., beta-alanine); and unnatural amino acids.

Amino acids can be named using the one-letter abbreviation or the three-letter abbreviation according to IUPAC (IUPAC-IUB Joint Commission on Biochemical Nomenclature. Nomenclature and Symbolism for Amino Acids and Peptides. Eur. J. Biochem. 138:9-37(1984). IUPAC-IUBMB JCBN Newsletter, 1999 http://www.chem.qmul.ac.uk/iubmb/newsletter/1999/item3.html), as shown in Table 3.

**Table 3.**

| Three-letter abbreviation | One-letter abbreviation | Amino acid name |
|---|---|---|
| Ala | A | Alanine |
| Arg | R | Arginine |
| Asn | N | Asparagine |
| Asp | D | Aspartic acid (Aspartate) |
| Cys | C | Cysteine |
| Gln | Q | Glutamine |
| Glu | E | Glutamic acid |
| Gly | G | Glycine |
| His | H | Histidine |
| Ile | I | Isoleucine |
| Leu | L | Leucine |
| Lys | K | Lysine |
| Met | M | Methionine |
| Phe | F | Phenylalanine |
| Pro | P | Proline |
| Ser | S | Serine |
| Thr | T | Threonine |
| Trp | W | Tryptophan |
| Tyr | Y | Tyrosine |
| Val | V | Valine |

The term "unnatural amino acid" comprises non-natura amino acids. Illustrative non-limitative examples of unnatural amino acids are summarized in Table 4:

**Table 4.**

| | |
|---|---|
| Aad | 2-Aminoadipic acid |
| bAad | 3-Aminoadipic acid |
| bAla | beta-Alanine, beta-Aminopropionic acid |
| Abu | 2-Aminobutyric acid |
| 4Abu | 4-Aminobutyric acid, piperidinic acid |
| Acp | 6-Aminocaproic acid |
| Ahe | 2-Aminoheptanoic acid |
| Aib | 2-Aminoisobutyric acid |
| bAib | 3-Aminoisobutyric acid |
| Apm | 2-Aminopimelic acid |
| Dbu | 2,4 Diaminobutyric acid |
| Des | Desmosine |
| Dpm | 2,2'-Diaminopimelic acid |
| Dpr | 2,3-Diaminopropionic acid |
| EtGly | N-Ethylglycine |
| EtAsn | N-Ethylasparagine |
| Hyl | Hydroxylysine |
| aHyl | allo-Hydroxylysine |
| 3Hyp | 3-Hydroxyproline |
| 4Hyp | 4-Hydroxyproline |
| Ide | Isodesmosine |
| alle | allo-Isoleucine |
| Nva | Norvaline |
| Nle | Norleucine |
| Orn | Ornithine |

In the context of the invention, the term "peptide" refers to polymers from at least two amino acids, formed by a condensation reaction, joining together through a peptide bond. Sequential peptide bonds with additional amino acids yield a peptide chain and the building block of proteins. Amino acids being part of a peptide are also called "residues". The terms "amino acid" and "residue" are used interchangeably.

A "mutant" protein is referred to a protein encoded by a gene with at least one mutation. "Single mutation" or "single mutant" refers to the substitution or change of one residue within the protein sequence by another residue. "Double mutation" or "double mutant" refers to the change or substitution of two residues by other residues within the protein sequence.

### DESCRIPTION OF EMBODIMENTS

Public sequence databases (Uniprot and NCBI) were scrutinized in terms of sequences having either a desired sequence pattern related to decarboxylase/dehydrogenase or suitable enzyme commission (EC) code if provided. The collected sequences were subjected to succinate compatibility by profiling their 3D binding site region in terms of protein-ligand interactions. The stability of the succinate-binding site interaction of each sequence was evaluated through molecular mechanics minimizations to determine if unbinding (e.g., protein-succinate interaction distorted) could be eventually observed, only retaining those where geometry interaction was stable. The retained sequences were selected based on visual inspection to determine the most compatible protein sequence solutions for experimental evaluation. This *in silico* analysis led to the unexpected identification of SD1 to SD6 enzymes using succinate as a substrate. The first *in vitro* activity screening showed 5-20% succinate converting activity for SD1, SD3, SD5 and SD6 (Fig. 1). Particularly, SD1 and SD5 were selected for further studies. Both SD1 and SD5 reduced succinate concentration and increased fumarate production *in vitro* under different experimental conditions (Fig. 2). SD1 also showed ability to produce malate (Fig. 27). The activity of SD1 was optimized in different expression systems, which showed up to 70% reduction in succinate levels (Fig. 3). The activity of different constructions of SD1 and SD5 enzymes was also measured by LC-MS/MS. The production of fumarate was assessed in all constructs (Fig. 4).

The capacity of the SD5 enzyme of reducing succinate was tested in a hepatic cell line. SD5 effectively reduced succinate concentration in the culture supernatant of HepG2 cells incubated with 20 µM succinate (Fig. 12) and in colonic cells (Fig. 14).

In a diabetes and obesity murine model, the intraperitoneal administration of SD1 prevented succinate levels restoration during fasting (Fig. 16). In diet-induced non-alcoholic fatty liver disease (NAFLD) model, the administration of SD1 ameliorated glucose tolerance (Fig. 17) SD1 and SD5 enzymes were found to decrease circulating succinate levels in diet-induced NAFLD (Fig. 18). SD1 and SD5 reduced disease activity (Fig. 19) and improved mucosal healing (Fig. 21) in male mice with dextran sodium sulfate-induced colitis. In a severe relapse colitis, 7 mg/kg of SD1 significantly reduced disease activity index (DAI), improved the colon weight-to-length ratio and reduced colon damage in female mice (Fig. 22).

The generated variants of SD1 and SD5 by semirational mutagenesis showed an improved capacity of reducing succinate compared to wild-type SD1 and SD5 *in vitro* (Fig. 25 and Fig. 26,). Variant SD1v489 was able t improve glucose tolerance and reduce lipid droplet *in vivo.*

Thus a first aspect of the invention refers to the *in vitro* use of a decarboxylase enzyme pertaining to carboxy-lyase EC 4.1.1. subclass for carrying out a biochemical enzymatic reaction involving said decarboxylase enzyme which converts succinate (substrate) to at least a product, thereby reducing the concentration of the substrate in comparison to the initial concentration of the substrate prior to carrying out the biochemical enzymatic reaction.

According to the first aspect of the present invention, the conversion of succinate (as a substrate of the enzymatic reaction) results in the formation of at least a product, thereby reducing the concentration of the substrate in comparison to the initial concentration of the substrate prior to carrying out the biochemical enzymatic reaction. The formation of at least one product may comprise at least one conversion step. For example, a product of said enzymatic reaction of reducing the concentration of the substrate, succinate, can be, without being limited to, fumarate, malate and/or propionate. It is noted that an exemplary method for determining whether the conversion of succinate has been effective, comprises the following steps:
(a) determining the levels of succinate prior to carrying out the biochemical enzymatic reaction, and
(b) determining the levels of succinate after carrying out the biochemical enzymatic reaction, wherein

If the level of b) is lower than the level of a), this is indicative that the biochemical enzymatic reaction has been effective. In the context of the present invention, the concentration of the substrate in comparison to the initial concentration of the substrate has been reduced if the concentration of step b) above is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more lower than the concentration of a).

The measurement of succinate levels by enzymatic activity *in vitro* can be performed by preferably incubating succinic acid with a buffer and the enzyme for 24 h at 37°C. Another reaction with succinic acid and the same buffer is performed but without the enzyme as a control. After that, the reaction is stopped with a 20-minute incubation at 80°C. The remaining succinate is then measured by any of the methods as described below.

Methods for succinate measurement or quantification in an isolated sample from a subject can be gas chromatography coupled to mass spectrometry (GC-MS), liquid chromatography tandem mass spectrometry (LC-MS/MS) (Lamy C, et al. Quantification of succinic acid levels, linked to succinate dehydrogenase (SDH) dysfunctions, by an automated and fully validated liquid chromatography tandem mass spectrometry method suitable for multi-matrix applications. J Chromatogr B Analyt Technol Biomed Life Sci. 2022 Jan 15;1189:123085. doi: 10.1016/j.jchromb.2021.123085.), colorimetric assay kits, fluorimetric assay kits or bioluminescent assay kits. For instance, a suitable bioluminiscent assay kit for succinate quantification can be Succinate-Glo^{™} JmjC Demethylase/Hydroxylase Assay. A suitable fluorimetric assay kit can be EnzyChrom^{™} Succinate Assay Kit.

Prior to succinate measurement in an isolated sample from a subject, a standard curve to estimate the amount of succinate can be generated.

It is noted that in an embodiment of the first aspect of the invention, succinate can be detected in any medium including isolated body fluids. Body fluids comprise for example blood, urine, stool, semen, tears, mucus, sweat, milk, cerebrospinal fluid and/or saliva/buccal swabs. These types of samples are routinely used in the clinical practice and a person skilled in the art will know how to identify the most appropriate means for their obtaining and preservation. Once a sample has been obtained, it may be used fresh, it may be frozen or preserved using appropriate means (e.g., as a formalin-fixed, paraffin-embedded tissue sample). Such biological samples can be taken around the time of diagnosis, before, during or after treatment (e.g. surgical resection).

Succinate can accumulate in the cytosol due to aerobic glycolysis, breakdown of the TCA cycle and anaplerosis. Said processes are involved in hypoxia, immune cell activation and tumorigenesis. Increased levels of cytosolic succinate can induce protein succinylation, stabilization of HIF-1α, epigenetic alterations and mitochondrial reactive oxygen species (ROS) production (Grimolizzi, F. and Arranz, L. (2018) Multiple faces of succinate beyond metabolism in blood. Haematologica 103,1586-1592; Murphy, M.P. and O'Neill, L.A.J. (2018) Krebs cycle reimagined: the emerging roles of succinate and itaconate as signal transducers. Cell 174, 780-784.) Succinate can also be released to the extracellular space via specific plasma membrane transporters and can be sensed by succinate receptor 1 (SUCNR1) (Fernández-Veledo S, Ceperuelo-Mallafré V, Vendrell J. Rethinking succinate: an unexpected hormone-like metabolite in energy homeostasis. Trends Endocrinol Metab. 2021 Sep;32(9):680-692. doi: 10.1016/j.tem.2021.06.003.)

In an embodiment of the first aspect of the invention, the enzyme is selected from the list consisting of cis-aconitate decarboxylase (SD1) of SEQ ID NO 1, ferulic acid decarboxylase 1 (SD5) of SEQ ID NO 2, or any of gallate decarboxylase of SEQ ID NO 3 (SD2), acetoacetate decarboxylase of SEQ ID NO 4 (SD3), methylmalonyl-coA decarboxylase of SEQ ID NO 5 (SD4) or arylmalonate decarboxylase of SEQ ID NO 6 (SD6), or a modified sequence having a percentage of sequence identity of at least 85% with any of sequences SEQ ID NO: 1 to 6 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

The capacity of reducing succinate by enzymes SD1 to SD6 *in vitro* is shown in Fig. 1.

Cis-aconitate decarboxylase (also named aconitate decarboxylase or cis-aconitic acid decarboxylase, EC: 4.1.1.6, SD1) is an enzyme related to the production of itaconic acid, which is produced from sugars, produced by *Aspergillus terreus.* It is encoded by cad1 gene, it has 490 residues of length, with an amino acid sequence corresponding of SEQ ID NO: 1 (UniProt. B3IUN8 CAD_ASPTE. Accessed 06.06.2024, retrieved from https://www.uniprot.org/uniprotkb/B3IUN8/entry).

SEQ ID NO: 1

MTKQSADSNAKSGVTSEICHWASNLATDDIPSDVLERAKYLILDGIACAWVGARVPWS

EKYVQATMSFEPPGACRVIGYGQKLGPVAAAMTNSAFIQATELDDYHSEAPLHSASIV

LPAVFAASEVLAEQGKTISGIDVILAAIVGFESGPRIGKAIYGSDLLNNGWHCGAVYGA

PAGALATGKLLGLTPDSMEDALGIACTQACGLMSAQYGGMVKRVQHGFAARNGLLG

GLLAHGGYEAMKGVLERSYGGFLKMFTKGNGREPPYKEEEVVAGLGSFWHTFTIRIK

LYACCGLVHGPVEAIENLQGRYPELLNRANLSNIRHVHVQLSTASNSHCGWIPEERPI

SSIAGQMSVAYILAVQLVDQQCLLSQFSEFDDNLERPEVWDLARKVTSSQSEEFDQD

GNCLSAGRVRIEFNDGSSITESVEKPLGVKEPMPNERILHKYRTLAGSVTDESRVKEIE

DLVLGLDRLTDISPLLELLNCPVKSPLV

Ferulic acid decarboxylase is an enzyme found in *Saccharomyces cerevisiae* (strain ATCC 204508/S288c) that catalyzes the reversible decarboxylation of aromatic carboxylic acids. Synonyms are phenacrylate decarboxylase, EC 4.1.1.102, SD5. It is encoded by FDC1 gene, with 503 residues of length. Ferulic acid decarboxylase amino acid sequence corresponds to SEQ ID NO: 2 (UniProt. Q03034.FDC1_YEAST. Accessed 06.06.2024, retrieved from https://www.uniprot.org/uniprotkb/Q03034/entry#function).

SEQ ID NO: 2

MRKLNPALEFRDFIQVLKDEDDLIEITEEIDPNLEVGAIMRKAYESHLPAPLFKNLKGAS

KDLFSILGCPAGLRSKEKGDHGRIAHHLGLDPKTTIKEIIDYLLECKEKEPLPPITVPVSS

APCKTHILSEEKIHLQSLPTPYLHVSDGGKYLQTYGMWILQTPDKKWTNWSIARGMVV

DDKHITGLVIKPQHIRQIADSWAAIGKANEIPFALCFGVPPAAILVSSMPIPEGVSESDY

VGAILGESVPVVKCETNDLMVPATSEMVFEGTLSLTDTHLEGPFGEMHGYVFKSQGH

PCPLYTVKAMSYRDNAILPVSNPGLCTDETHTLIGSLVATEAKELAIESGLPILDAFMPY

EAQALWLILKVDLKGLQALKTTPEEFCKKVGDIYFRTKVGFIVHEIILVADDIDIFNFKEVI

WAYVTRHTPVADQMAFDDVTSFPLAPFVSQSSRSKTMKGGKCVTNCIFRQQYERSF

DYITCNFEKGYPKGLVDKVNENWKRYGYK

Gallate decarboxylase is an enzyme related to tannin degradation. It is found in *Lactiplantibacillus plantarum* (strain ATCC BAA-793 / NCIMB 8826 / WCFS1) *(Lactobacillus plantarum)* and it catalyzes the decarboxylation of gallic acid and protocatechuic acid to pyrogallol and catechol, respectively. Synonyms of gallate decarboxylase are gallate decarboxylase subunit and prototechuate decarboxylase, EC 4.1.1.59, SD2. It is encoded by IpdC gene, having 490 residues of length and an amino acid sequence corresponding to SEQ ID NO: 3 (UniProt. F9US27.LPDC_LACPL. Accessed 06.06.2024, retrieved from https://www.uniprot.org)/uniprotkb/F9US27/entry#function.)

SEQ ID NO: 3

MAEQPWDLRRVLDEIKDDPKNYHETDVEVDPNAELSGVYRYIGAGGTVQRPTQEGP

AMMFNNVKGFPDTRVLTGLMASRRRVGKMFHHDYQTLGQYLNEAVSNPVAPETVAE

ADAPAHDVVYKATDEGFDIRKLVAAPTNTPQDAGPYITVGVVFGSSMDKSKSDVTIHR

MVLEDKDKLGIYIMPGGRHIGAFAEEYEKANKPMPITINIGLDPAITIGATFEPPTTPFGY

NELGVAGAIRNQAVQLVDGVTVDEKAIARSEYTLEGYIMPNERIQEDINTHTGKAMPEF

PGYDGDANPALQVIKVTAVTHRKNAIMQSVIGPSEEHVSMAGIPTEASILQLVNRAIPG

KVTNVYNPPAGGGKLMTIMQIHKDNEADEGIQRQAALLAFSAFKELKTVILVDEDVDIF

DMNDVIWTMNTRFQADQDLMVLSGMRNHPLDPSERPQYDPKSIRFRGMSSKLVIDG

TVPFDMKDQFERAQFMKVADWEKYLK

Acetoacetate decarboxylase (also AAD, AADase, ADC, EC: 4.1.1.4 or SD3) is found in *Clostridium acetobutylicum* ((strain ATCC 824 / DSM 7921 JCM 1419 / IAM 19013 / LMG 5710 / NBRC 13948 / NRRL B-527 / VKM B-1787 / 2291 / W). It catalyzes the conversion of acetoacetate to acetone and carbon dioxide. It is encoded by adc gene, having 244 residues of length and corresponds to SEQ ID NO: 4. (UniProt. P23670 ADC_CLOAB. Accessed 06.06.2024, retrieved from https://www.uniprot.org/uniprotkb/P23670/entry#function.)

SEQ ID NO: 4

MLKDEVIKQISTPLTSPAFPRGPYKFHNREYFNIVYRTDMDALRKVVPEPLEIDEPLVR

FEIMAMHDTSGLGCYTESGQAIPVSFNGVKGDYLHMMYLDNEPAIAVGRELSAYPKK

LGYPKLFVDSDTLVGTLDYGKLRVATATMGYKHKALDANEAKDQICRPNYMLKIIPNY

DGSPRICELINAKITDVTVHEAWTGPTRLQLFDHAMAPLNDLPVKEIVSSSHILADIILPR

AEVIYDYLK

Methylmalonyl-coA decarboxylase is an enzyme of 261 residues of length produced by *Escherichia coli* (strain K12). This enzyme catalyzes the decarboxylation of (R)-methylmalonyl-CoA to propionyl-CoA. Synonyms of Methylmalonyl-coA decarboxylase are transcarboxylase, EC 4.1.1.- or SD4. It is encoded by scpB gene, having an amino acid sequence corresponding to SEQ ID NO: 5 (UniProt. P52045.scpb_ECOLI. Accessed 06.06.2024, retrieved from https://www.uniprot.org/uniprotkb/P52045/entry#names and taxonomy).

SEQ ID NO: 5

MSYQYVNVVTINKVAVIEFNYGRKLNALSKVFIDDLMQALSDLNRPEIRCIILRAPSGSK

VFSAGHDIHELPSGGRDPLSYDDPLRQITRMIQKFPKPIISMVEGSVWGGAFEMIMSS

DLIIAASTSTFSMTPVNLGVPYNLVGIHNLTRDAGFHIVKELIFTASPITAQRALAVGILN

HVVEVEELEDFTLQMAHHISEKAPLAIAVIKEELRVLGEAHTMNSDEFERIQGMRRAVY

DSEDYQEGMNAFLEKRKPNFVGH

Arylmalonate decarboxylase is an enzyme that catalyzes the conversion of 2-aryl-2-methylmalonate into 2-arylpropionate. Synonyms are AMDase, EC 4.1.1.76 or SD6. It is found in *Bordetella bronchiseptica (Alcaligenes bronchisepticus).* It has a length of 240 residues and its sequence corresponds to SEQ ID NO: 6. (UniProt. Q05115.AMDA_BORBO. Accessed 06.06.2024, retrieved from https://www.uniprot.org/uniprotkb/Q05115/entry#function).

SEQ ID NO: 6

MQQASTPTIGMIVPPAAGLVPADGARLYPDLPFIASGLGLGSVTPEGYDAVIESVVDH

ARRLQKQGAAVVSLMGTSLSFYRGAAFNAALTVAMREATGLPCTTMSTAVLNGLRAL

GVRRVALATAYIDDVNERLAAFLAEESLVPTGCRSLGITGVEAMARVDTATLVDLCVR

AFEAAPDSDGILLSCGGLLTLDAIPEVERRLGVPVVSSSPAGFWDAVRLAGGGAKARP

GYGRLFDES

In another embodiment of the first aspect of the invention, the enzyme is selected from the list consisting of cis-aconitate decarboxylase (SD1) of SEQ ID NO 1 and ferulic acid decarboxylase 1 (SD5) of SEQ ID NO 2, or a modified sequence having a percentage of sequence identity of at least 85% with any of sequences SEQ ID NO 1 or 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the first aspect of the invention, the enzyme is cis-aconitate decarboxylase (SD1) of SEQ ID NO 1, or a modified sequence having a percentage of sequence identity of at least 85% with SEQ ID NO 1 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

Particularly, the enzyme is cis-aconitate decarboxylase (SD1) of SEQ ID NO 1 or a modified sequence comprising at least one or more mutations with respect to SEQ ID NO 1, wherein the modified sequence is characterized by comprising the amino acid sequence represented by SEQ ID NO 1 with at least one substitution in the corresponding amino acid position of the modified amino acid sequence with respect to SEQ ID NO 1 selected from the list of Table 2. These modified sequences are capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

**Table 2.**

| Amino acid of SEQ ID NO 1 | Position in SEQ ID NO 1 | Amino acid of the modified sequence |
|---|---|---|
| L | 110 | W |
| H | 168 | A |
| H | 168 | K |
| K | 217 | A |
| K | 217 | Q |
| K | 288 | A |
| K | 288 | Q |
| G | 294 | A |
| H | 336 | A |
| H | 336 | K |

A mutation is a change in the nucleotide sequence of a region of a genome. The replacement of one nucleotide by another one is referred as to point mutation. Other mutations may involve insertion or deletion of one or a few nucleotides. Mutations can change the sequence of a triplet codon. A non-synonymous or missense change leads to a codon specifying a different amino acid from the unmutated codon.

In another embodiment of the first aspect of the invention, optionally in combination with any of the previously indicated embodiments of the first aspect of the invention, the modified sequence is characterized by comprising the amino acid sequence represented by SEQ ID NO 1 with at least one substitution of the amino acid in the 110 position, L (leucine, according to the one-letter abbreviation), of the modified amino acid sequence with respect to SEQ ID NO: 1 by W (tryptophan), as shown in Table 2. In another embodiment, the modified sequence is characterized by comprising the amino acid sequence represented by SEQ ID NO 1 with at least one substitution of the amino acid in the 168 position, H (histidine), of the modified amino acid sequence with respect to SEQ ID NO: 1 by A (alanine) or K (lysine), as shown in Table 2. In another embodiment, the modified sequence is characterized by comprising the amino acid sequence represented by SEQ ID NO 1 with at least one substitution of the amino acid in the 217 position, K (lysine), of the modified amino acid sequence with respect to SEQ ID NO: 1 by A (alanine) or Q (glutamine), as shown in Table 2. In another embodiment, the modified sequence is characterized by comprising the amino acid sequence represented by SEQ ID NO 1 with at least one substitution of the amino acid in the 288 position, K (lysine), of the modified amino acid sequence with respect to SEQ ID NO: 1 by A (alanine) or Q (glutamine), as shown in Table 2. In another embodiment, the modified sequence is characterized by comprising the amino acid sequence represented by SEQ ID NO 1 with at least one substitution of the amino acid in the 294 position, G (glycine), of the modified amino acid sequence with respect to SEQ ID NO: 1 by A (alanine) as shown in Table 2. In another embodiment, the modified sequence is characterized by comprising the amino acid sequence represented by SEQ ID NO 1 with at least the substitution of the amino acid sequence in the 336 position, H (histidine), of the modified amino acid sequence with respect to SEQ ID NO: 1 by A (alanine), or K (lysine) as shown in Table 2.

In another embodiment of the first aspect of the invention, optionally in combination with any of the previously indicated embodiments of the first aspect of the invention, the enzyme is cis-aconitate decarboxylase (SD1) of SEQ ID NO 1 or a modified sequence comprising at least two or more mutations with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction. In another embodiment, the enzyme is cis-aconitate decarboxylase (SD1) of SEQ ID NO 1 or a modified sequence comprising at least three or more mutations with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction. In another embodiment of the first aspect of the invention, optionally in combination with any of the previously indicated embodiments of the first aspect of the invention, the enzyme is cis-aconitate decarboxylase (SD1) of SEQ ID NO 1 or a modified sequence comprising at least four or more mutations with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction. In another embodiment, the enzyme is cis-aconitate decarboxylase (SD1) of SEQ ID NO 1 or a modified sequence comprising at least five or more mutations with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction. In another embodiment, the enzyme is cis-aconitate decarboxylase (SD1) of SEQ ID NO 1 or a modified sequence comprising at least all mutations with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction. The capacity of succinate degradation by SD1 mutants is provided in Fig. 25.

In another embodiment of the first aspect of the invention, optionally in combination with any of the previously indicated embodiments of the first aspect of the invention, the enzyme is ferulic acid decarboxylase 1 (SD5) of SEQ ID NO 2 or a modified sequence having a percentage of sequence identity of at least 85% with SEQ ID NO 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the first aspect of the invention, optionally in combination with any of the previously indicated embodiments of the first aspect of the invention, the enzyme is ferulic acid decarboxylase 1 (SD5) of SEQ ID NO 2 or a modified sequence thereof characterized by comprising at least one or more mutations with respect to SEQ ID NO 2, wherein the modified sequence is characterized by comprising the amino acid sequence represented by SEQ ID NO 2 with at least one substitution in the corresponding amino acid position of the modified amino acid sequence with respect to SEQ ID NO 2 selected from the list of Table 5. The capacity of reducing succinate by SD5 mutants is shown in Fig. 26.

**Table 5. Single mutations of SD5**

| Amino acid of SEQ ID NO 2 | Position in SEQ ID NO 2 | Amino acid of the modified sequence |
|---|---|---|
| H | 145 | A |
| H | 145 | D |
| H | 145 | F |
| H | 145 | G |
| H | 145 | K |
| H | 145 | L |
| H | 145 | N |
| H | 145 | P |
| H | 145 | S |
| Y | 156 | C |
| Y | 156 | D |
| Y | 156 | F |
| Y | 156 | G |
| Y | 156 | H |
| Y | 156 | L |
| Y | 156 | P |
| Y | 156 | S |
| I | 173 | C |
| I | 173 | E |
| I | 173 | F |
| I | 173 | G |
| I | 173 | K |
| I | 173 | P |
| I | 173 | T |
| I | 173 | V |
| L | 187 | A |
| L | 187 | E |
| L | 187 | F |
| L | 187 | G |
| L | 187 | M |
| L | 187 | P |
| L | 187 | R |
| L | 187 | T |
| I | 189 | A |
| I | 189 | D |
| I | 189 | F |
| I | 189 | G |
| I | 189 | K |
| I | 189 | P |
| I | 189 | T |
| I | 189 | V |
| P | 191 | A |
| P | 191 | E |
| P | 191 | G |
| P | 191 | K |
| P | 191 | L |
| P | 191 | Q |
| P | 191 | S |
| P | 191 | Y |
| Q | 192 | A |
| Q | 192 | E |
| Q | 192 | G |
| Q | 192 | H |
| Q | 192 | L |
| Q | 192 | P |
| Q | 192 | S |
| Q | 192 | Y |
| S | 227 | A |
| S | 227 | E |
| S | 227 | F |
| S | 227 | G |
| S | 227 | K |
| S | 227 | P |
| S | 227 | T |
| S | 227 | V |
| P | 229 | A |
| P | 229 | E |
| P | 229 | G |
| P | 229 | K |
| P | 229 | S |
| P | 229 | T |
| P | 229 | V |
| P | 229 | Y |
| M | 286 | A |
| M | 286 | E |
| M | 286 | F |
| M | 286 | G |
| M | 286 | H |
| M | 286 | P |
| M | 286 | S |
| M | 286 | V |
| M | 286 | Y |
| H | 287 | A |
| H | 287 | E |
| H | 287 | G |
| H | 287 | M |
| H | 287 | P |
| H | 287 | R |
| H | 287 | T |
| H | 287 | Y |
| F | 291 | A |
| F | 291 | E |
| F | 291 | G |
| F | 291 | H |
| F | 291 | L |
| F | 291 | P |
| F | 291 | S |
| F | 291 | Y |
| C | 298 | A |
| C | 298 | E |
| C | 298 | G |
| C | 298 | K |
| C | 298 | M |
| C | 298 | N |
| C | 298 | P |
| C | 298 | S |
| C | 298 | Y |
| S | 317 | C |
| S | 317 | D |
| S | 317 | F |
| S | 317 | G |
| S | 317 | P |
| S | 317 | R |
| S | 317 | T |
| S | 317 | V |
| P | 319 | A |
| P | 319 | E |
| P | 319 | F |
| P | 319 | G |
| P | 319 | K |
| P | 319 | Q |
| P | 319 | S |
| P | 319 | V |
| D | 324 | A |
| D | 324 | E |
| D | 324 | G |
| D | 324 | K |
| D | 324 | N |
| D | 324 | P |
| D | 324 | S |
| D | 324 | V |
| D | 324 | Y |
| E | 325 | A |
| E | 325 | D |
| E | 325 | G |
| E | 325 | K |
| E | 325 | L |
| E | 325 | P |
| E | 325 | Q |
| E | 325 | S |
| E | 325 | Y |
| T | 326 | A |
| T | 326 | E |
| T | 326 | F |
| T | 326 | G |
| T | 326 | I |
| T | 326 | K |
| T | 326 | N |
| T | 326 | P |
| T | 326 | S |
| I | 330 | A |
| I | 330 | E |
| I | 330 | G |
| I | 330 | L |
| I | 330 | P |
| I | 330 | R |
| I | 330 | T |
| I | 330 | W |
| V | 334 | A |
| V | 334 | E |
| V | 334 | F |
| V | 334 | G |
| V | 334 | I |
| V | 334 | P |
| V | 334 | R |
| V | 334 | T |
| A | 360 | C |
| A | 360 | E |
| A | 360 | F |
| A | 360 | G |
| A | 360 | P |
| A | 360 | R |
| A | 360 | T |
| A | 360 | V |
| L | 361 | C |
| L | 361 | D |
| L | 361 | F |
| L | 361 | G |
| L | 361 | P |
| L | 361 | R |
| L | 361 | T |
| L | 361 | V |
| V | 395 | A |
| V | 395 | E |
| V | 395 | F |
| V | 395 | G |
| V | 395 | H |
| V | 395 | L |
| V | 395 | P |
| V | 395 | S |
| V | 395 | Y |
| F | 397 | A |
| F | 397 | D |
| F | 397 | G |
| F | 397 | L |
| F | 397 | N |
| F | 397 | P |
| F | 397 | R |
| F | 397 | S |
| F | 397 | Y |
| I | 398 | A |
| I | 398 | D |
| I | 398 | F |
| I | 398 | G |
| I | 398 | L |
| I | 398 | N |
| I | 398 | P |
| I | 398 | R |
| I | 398 | S |
| F | 440 | C |
| F | 440 | D |
| F | 440 | G |
| F | 440 | I |
| F | 440 | P |
| F | 440 | R |
| F | 440 | T |
| F | 440 | Y |
| P | 441 | A |
| P | 441 | D |
| P | 441 | G |
| P | 441 | Q |
| P | 441 | R |
| P | 441 | S |
| P | 441 | V |
| P | 441 | Y |
| L | 442 | A |
| L | 442 | D |
| L | 442 | F |
| L | 442 | G |
| L | 442 | I |
| L | 442 | P |
| L | 442 | R |
| L | 442 | T |
| A | 443 | C |
| A | 443 | D |
| A | 443 | F |
| A | 443 | G |
| A | 443 | I |
| A | 443 | P |
| A | 443 | R |
| A | 443 | T |
| A | 443 | V |
| S | 447 | A |
| S | 447 | D |
| S | 447 | G |
| S | 447 | K |
| S | 447 | L |
| S | 447 | P |
| S | 447 | T |
| S | 447 | Y |

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 145 position, H (histidine), of the modified amino acid sequence with respect to SEQ ID NO 2 by A (alanine), D (aspartate), F (phenylalanine), G (glycine), K (lysine), L (leucine), N (asparagine), P (proline) or S (serine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 156 position, Y (tyrosine), of the modified amino acid sequence with respect to SEQ ID NO 2 by C (cysteine), D (aspartate), F (phenylalanine), G (glycine), H (histidine), L (leucine), P (proline) or S (serine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 173 position, I (isoleucine), of the modified amino acid sequence with respect to SEQ ID NO 2 by C (cysteine), E (glutamate), F (phenylalanine), G (glycine), K (lysine), P (proline), T (threonine) or V (valine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 187 position, L (leucine), of the modified amino acid sequence with respect to SEQ ID NO 2 by A (alanine), E (glutamate), F (phenylalanine), G (glycine), M (methionine), P (proline), R (arginine) or T (threonine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 189 position, I (isoleucine), of the modified amino acid sequence with respect to SEQ ID NO 2 by A (alanine), D (aspartate), F (phenylalanine), G (glycine), K (lysine), P (proline), T (threonine) or V (valine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 191 position, P (proline), of the modified amino acid sequence with respect to SEQ ID NO 2 by A (alanine), E (glutamate), G (glycine), K (lysine), L (leucine), Q (glutamine), S (serine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 192 position, Q (glutamine), of the modified amino acid sequence with respect to SEQ ID NO 2 by A (alanine), E (glutamate), G (glycine), H (histidine), L (leucine), P (proline), S (serine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 227 position, S (serine), of the modified amino acid sequence with respect to SEQ ID NO 2 by A (alanine), E (glutamate), F (phenylalanine), G (glycine), K (lysine), P (proline), T (threonine) or V (valine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 229 position, P (proline), of the modified amino acid sequence with respect to SEQ ID NO 2 by A (alanine), E (glutamate), G (glycine), K (lysine), S (serine), T (threonine), V (valine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 286 position, M (methionine), of the modified amino acid sequence with respect to SEQ ID NO 2 by A (alanine), E (glutamate), F (phenylalanine), G (glycine), H (histidine), P (proline), S (serine), V (valine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 287 position, H (histidine), of the modified amino acid sequence with respect to SEQ ID NO 2 by A (alanine), E (glutamate), G (glycine), M (methionine), P (proline), R (arginine), T (threonine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 291 position, F (phenylalanine), of the modified amino acid sequence with respect to SEQ ID NO 2 by A (alanine), E (glutamate), G (glycine), H (histidine), L (leucine), P (proline), S (serine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 298 position, C (cysteine), of the modified amino acid sequence with respect to SEQ ID NO 2 by A (alanine), E (glutamate), G (glycine), K (lysine), M (methionine), N (asparagine), P (proline), S (serine), or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 317 position, S (serine), of the modified amino acid sequence with respect to SEQ ID NO 2 by C (cysteine), D (aspartate), F (phenylalanine), G (glycine), P (proline), R (arginine), T (threonine) or V (valine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 319 position, P (proline), of the modified amino acid sequence with respect to SEQ ID NO 2 by A (alanine), E (glutamate), F (phenylalanine), G (glycine), K (lysine), Q (glutamine), S (serine) or V (valine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 324 position, D (aspartate), of the modified amino acid sequence with respect to SEQ ID NO 2 by A (alanine), E (glutamate), G (glycine), K (lysine), N (asparagine), P (proline), S (serine), V (valine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 325 position, E (glutamate), of the modified amino acid sequence with respect to SEQ ID NO 2 by A (alanine), D (aspartate), G (glycine), K (lysine), L (leucine), P (proline), Q (glutamine), S (serine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 326 position, T (threonine), of the modified amino acid sequence with respect to SEQ ID NO 2 by A (alanine), E (glutamate), F (phenylalanine), G (glycine), I (isoleucine), K (lysine), N (asparagine), P (proline) or S (serine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 330 position, I (isoleucine), of the modified amino acid sequence with respect to SEQ ID NO 2 by A (alanine), E (glutamate), G (glycine), L (leucine), P (proline), R (arginine), T (threonine) or W (tryptophan), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 334 position, V (valine), of the modified amino acid sequence with respect to SEQ ID NO 2 by A (alanine), E (glutamate), F (phenylalanine), G (glycine), I (isoleucine), P (proline), R (arginine) or T (threonine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 360 position, A (alanine), of the modified amino acid sequence with respect to SEQ ID NO 2 by C (cysteine), E (glutamate), F (phenylalanine), G (glycine), P (proline), R (arginine), T (threonine) or V (valine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 361 position, L (leucine), of the modified amino acid sequence with respect to SEQ ID NO 2 by C (cysteine), D (aspartate), F (phenylalanine), G (glycine), P (proline), R (arginine), T (threonine) or V (valine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 395 position, V (valine), of the modified amino acid sequence with respect to SEQ ID NO 2 by A (alanine), E (glutamate), F (phenylalanine), G (glycine), H (histidine), L (leucine), P (proline), S (serine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 397 position, F (phenylalanine), of the modified amino acid sequence with respect to SEQ ID NO 2 by A (alanine), D (aspartate), G (glycine), L (leucine), N (asparagine), P (proline), R (arginine), S (serine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 398 position, I (isoleucine), of the modified amino acid sequence with respect to SEQ ID NO 2 by A (alanine), D (aspartate), F (phenylalanine), G (glycine), L (leucine), N (asparagine), P (proline), R (arginine) or S (serine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 440 position, F (phenylalanine), of the modified amino acid sequence with respect to SEQ ID NO 2 by C (cysteine), D (aspartate), G (glycine), I (isoleucine), P (proline), R (arginine), T (threonine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 441 position, P (proline), of the modified amino acid sequence with respect to SEQ ID NO 2 by A (alanine), D (aspartate), G (glycine), Q (glutamine), R (arginine), S (serine), V (valine) or Y (tyrosine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 442 position, L (leucine), of the modified amino acid sequence with respect to SEQ ID NO 2 by A (alanine), D (aspartate), F (phenylalanine), G (glycine), I (isoleucine), P (proline), R (arginine) or T (threonine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 443 position, A (alanine), of the modified amino acid sequence with respect to SEQ ID NO 2 by C (cysteine), D (aspartate), F (phenylalanine), G (glycine), I (isoleucine), P (proline), R (arginine), T (threonine) or V (valine), as described in Table 5.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least the substitution in the 447 position, S (serine), of the modified amino acid sequence with respect to SEQ ID NO 2 by A (alanine), D (aspartate), G (glycine), K (lysine), L (leucine), P (proline), T (threonine) or Y (tyrosine), as described in Table 5.

In another embodiment of the first aspect of the invention, optionally in combination with any of the previously indicated embodiments of the first aspect of the invention, the modified sequence of SD5 is characterized by comprising at least 2, 3, 4, 5, 6, 7, 8, 9, 10, or more mutations with respect to SEQ ID NO 2 selected from the list of mutations indicated in Table 5, and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution in the corresponding amino acid positions of the modified amino acid sequence with respect to SEQ ID NO 2 selected from the list of mutations indicated in Table 6.

**Table 6. Double mutations of SD5**

| Position in SEQ ID NO 2 | Amino acid of the modified sequence | Position in SEQ ID NO 2 | Amino acid of the modified sequence |
|---|---|---|---|
| 145 | N | 286 | F |
| 145 | N | 317 | L |
| 145 | W | 286 | H |
| 145 | W | 317 | T |
| 156 | A | 326 | H |
| 156 | H | 326 | H |
| 156 | L | 319 | V |
| 156 | W | 319 | T |
| 156 | W | 326 | P |
| 187 | H | 286 | H |
| 187 | H | 291 | S |
| 187 | H | 298 | Y |
| 187 | P | 298 | H |
| 187 | P | 298 | Q |
| 187 | P | 298 | R |
| 187 | Q | 286 | F |
| 187 | R | 286 | H |
| 187 | R | 291 | D |
| 187 | R | 291 | N |
| 187 | R | 298 | F |
| 187 | R | 298 | Q |
| 189 | H | 291 | S |
| 189 | Q | 291 | S |
| 189 | Q | 291 | T |
| 191 | G | 298 | Q |
| 191 | G | 397 | A |
| 191 | H | 298 | Y |
| 191 | H | 397 | M |
| 229 | K | 330 | K |
| 229 | S | 330 | M |
| 229 | S | 398 | S |
| 286 | H | 298 | F |
| 286 | H | 317 | T |
| 287 | L | 442 | T |
| 287 | R | 447 | R |
| 287 | T | 442 | Y |
| 287 | Y | 447 | R |
| 319 | G | 326 | H |
| 319 | S | 324 | S |
| 319 | T | 326 | P |
| 319 | V | 324 | M |
| 319 | V | 324 | Q |
| 324 | N | 326 | G |
| 330 | M | 398 | S |
| 330 | M | 398 | Y |
| 330 | V | 398 | R |
| 334 | R | 361 | R |
| 334 | R | 361 | W |
| 334 | T | 361 | R |
| 442 | T | 447 | G |

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 145 position of the modified amino acid sequence with respect to SEQ ID NO 2 by N (asparagine) and the substitution in 286 position of the modified amino acid sequence with respect to SEQ ID NO 2 by F (phenylalanine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 145 position of the modified amino acid sequence with respect to SEQ ID NO 2 by N (asparagine) and the substitution in 317 position of the modified amino acid sequence with respect to SEQ ID NO 2 by L (leucine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 145 position of the modified amino acid sequence with respect to SEQ ID NO 2 by W (tryptophan) and the substitution in 286 position of the modified amino acid sequence with respect to SEQ ID NO 2 by H (histidine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 145 position of the modified amino acid sequence with respect to SEQ ID NO 2 by W (tryptophan) and the substitution in 317 position of the modified amino acid sequence with respect to SEQ ID NO 2 by T (threonine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 156 position of the modified amino acid sequence with respect to SEQ ID NO 2 by A (alanine) and the substitution in 326 position of the modified amino acid sequence with respect to SEQ ID NO 2 by H (histidine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 156 position of the modified amino acid sequence with respect to SEQ ID NO 2 by H (histidine) and the substitution in 326 position of the modified amino acid sequence with respect to SEQ ID NO 2 by H (histidine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 156 position of the modified amino acid sequence with respect to SEQ ID NO 2 by L (leucine) and the substitution in 319 position of the modified amino acid sequence with respect to SEQ ID NO 2 by V (valine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 156 position of the modified amino acid sequence with respect to SEQ ID NO 2 by W (tryptophan) and the substitution in 319 position of the modified amino acid sequence with respect to SEQ ID NO 2 by T (threonine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutations with respect to SEQ ID NO 2, wherein the modified sequence is characterized by comprising the amino acid sequence represented by SEQ ID NO 2 with at least one double substitution comprising the substitution in 156 position of the modified amino acid sequence with respect to SEQ ID NO 2 by W (tryptophan) and the substitution in 326 position of the modified amino acid sequence with respect to SEQ ID NO 2 by P (proline), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by H (histidine) and the substitution in 286 position of the modified amino acid sequence with respect to SEQ ID NO 2 by H (histidine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by H (histidine) and the substitution in 291 position of the modified amino acid sequence with respect to SEQ ID NO 2 by H (serine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by H (histidine) and the substitution in 298 position of the modified amino acid sequence with respect to SEQ ID NO 2 by Y (tyrosine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by P (proline) and the substitution in 298 position of the modified amino acid sequence with respect to SEQ ID NO 2 by H (histidine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by P (proline) and the substitution in 298 position of the modified amino acid sequence with respect to SEQ ID NO 2 by Q (glutamine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by P (proline) and the substitution in 298 position of the modified amino acid sequence with respect to SEQ ID NO 2 by R (arginine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by Q (glutamine) and the substitution in 286 position of the modified amino acid sequence with respect to SEQ ID NO 2 by F (phenylalanine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by R (arginine) and the substitution in 286 position of the modified amino acid sequence with respect to SEQ ID NO 2 by H (histidine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by R (arginine) and the substitution in 291 position of the modified amino acid sequence with respect to SEQ ID NO 2 by D (glutamate), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by R (arginine) and the substitution in 291 position of the modified amino acid sequence with respect to SEQ ID NO 2 by N (asparagine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by R (arginine) and the substitution in 298 position of the modified amino acid sequence with respect to SEQ ID NO 2 by F (phenylalanine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 187 position of the modified amino acid sequence with respect to SEQ ID NO 2 by R (arginine) and the substitution in 298 position of the modified amino acid sequence with respect to SEQ ID NO 2 by Q (glutamine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least the double substitution comprising the substitution in 189 position of the modified amino acid sequence with respect to SEQ ID NO 2 by H (histidine) and the substitution in 291 position of the modified amino acid sequence with respect to SEQ ID NO 2 by S (serine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 189 position of the modified amino acid sequence with respect to SEQ ID NO 2 by Q (glutamine) and the substitution in 291 position of the modified amino acid sequence with respect to SEQ ID NO 2 by S (serine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 189 position of the modified amino acid sequence with respect to SEQ ID NO 2 by Q (glutamine) and the substitution in 291 position of the modified amino acid sequence with respect to SEQ ID NO 2 by T (threonine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 191 position of the modified amino acid sequence with respect to SEQ ID NO 2 by G (glycine) and the substitution in 298 position of the modified amino acid sequence with respect to SEQ ID NO 2 by Q (glutamine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 191 position of the modified amino acid sequence with respect to SEQ ID NO 2 by G (glycine) and the substitution in 397 position of the modified amino acid sequence with respect to SEQ ID NO 2 by A (alanine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 191 position of the modified amino acid sequence with respect to SEQ ID NO 2 by H (histidine) and the substitution in 298 position of the modified amino acid sequence with respect to SEQ ID NO 2 by Y (tyrosine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 191 position of the modified amino acid sequence with respect to SEQ ID NO 2 by H (histidine) and the substitution in 397 position of the modified amino acid sequence with respect to SEQ ID NO 2 by M (methionine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 229 position of the modified amino acid sequence with respect to SEQ ID NO 2 by K (lysine) and the substitution in 330 position of the modified amino acid sequence with respect to SEQ ID NO 2 by K (lysine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 229 position of the modified amino acid sequence with respect to SEQ ID NO 2 by S (serine) and the substitution in 330 position of the modified amino acid sequence with respect to SEQ ID NO 2 by M (methionine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 229 position of the modified amino acid sequence with respect to SEQ ID NO 2 by S (serine) and the substitution in 398 position of the modified amino acid sequence with respect to SEQ ID NO 2 by S (serine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 286 position of the modified amino acid sequence with respect to SEQ ID NO 2 by H (histidine) and the substitution in 298 position of the modified amino acid sequence with respect to SEQ ID NO 2 by F (phenylalanine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 286 position of the modified amino acid sequence with respect to SEQ ID NO 2 by H (histidine) and the substitution in 317 position of the modified amino acid sequence with respect to SEQ ID NO 2 by T (threonine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 287 position of the modified amino acid sequence with respect to SEQ ID NO 2 by L (leucine) and the substitution in 442 position of the modified amino acid sequence with respect to SEQ ID NO 2 by T (threonine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 287 position of the modified amino acid sequence with respect to SEQ ID NO 2 by R (arginine) and the substitution in 447 position of the modified amino acid sequence with respect to SEQ ID NO 2 by R (arginine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 287 position of the modified amino acid sequence with respect to SEQ ID NO 2 by T (threonine) and the substitution in 442 position of the modified amino acid sequence with respect to SEQ ID NO 2 by Y (tyrosine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 287 position of the modified amino acid sequence with respect to SEQ ID NO 2 by Y (tyrosine) and the substitution in 447 position of the modified amino acid sequence with respect to SEQ ID NO 2 by R (arginine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 319 position of the modified amino acid sequence with respect to SEQ ID NO 2 by G (glycine) and the substitution in 326 position of the modified amino acid sequence with respect to SEQ ID NO 2 by H (histidine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 319 position of the modified amino acid sequence with respect to SEQ ID NO 2 by S (serine) and the substitution in 324 position of the modified amino acid sequence with respect to SEQ ID NO 2 by S (serine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 319 position of the modified amino acid sequence with respect to SEQ ID NO 2 by T (threonine) and the substitution in 326 position of the modified amino acid sequence with respect to SEQ ID NO 2 by P (proline), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 319 position of the modified amino acid sequence with respect to SEQ ID NO 2 by V (valine) and the substitution in 324 position of the modified amino acid sequence with respect to SEQ ID NO 2 by M (methionine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 319 position of the modified amino acid sequence with respect to SEQ ID NO 2 by V (valine) and the substitution in 324 position of the modified amino acid sequence with respect to SEQ ID NO 2 by Q (glutamine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 324 position of the modified amino acid sequence with respect to SEQ ID NO 2 by N (asparagine) and the substitution in 326 position of the modified amino acid sequence with respect to SEQ ID NO 2 by G (glycine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 330 position of the modified amino acid sequence with respect to SEQ ID NO 2 by M (methionine) and the substitution in 398 position of the modified amino acid sequence with respect to SEQ ID NO 2 by S (serine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 330 position of the modified amino acid sequence with respect to SEQ ID NO 2 by M (methionine) and the substitution in 398 position of the modified amino acid sequence with respect to SEQ ID NO 2 by Y (tyrosine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 330 position of the modified amino acid sequence with respect to SEQ ID NO 2 by V (valine) and the substitution in 398 position of the modified amino acid sequence with respect to SEQ ID NO 2 by R (arginine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutations with respect to SEQ ID NO 2, wherein the modified sequence is characterized by comprising the amino acid sequence represented by SEQ ID NO 2 with at least one double substitution comprising the substitution in 334 position of the modified amino acid sequence with respect to SEQ ID NO 2 by R (arginine) and the substitution in 361 position of the modified amino acid sequence with respect to SEQ ID NO 2 by R (arginine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 334 position of the modified amino acid sequence with respect to SEQ ID NO 2 by R (arginine) and the substitution in 361 position of the modified amino acid sequence with respect to SEQ ID NO 2 by W (tryptophan), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 334 position of the modified amino acid sequence with respect to SEQ ID NO 2 by T (threonine) and the substitution in 361 position of the modified amino acid sequence with respect to SEQ ID NO 2 by R (arginine), as shown in Table 6.

In another embodiment of the first aspect of the invention, optionally in combination with any of the previously indicated embodiments of the first aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution comprising the substitution in 442 position of the modified amino acid sequence with respect to SEQ ID NO 2 by T (threonine) and the substitution in 447 position of the modified amino acid sequence with respect to SEQ ID NO 2 by G (glycine), as shown in Table 6.

In another embodiment of the first aspect of the invention, optionally in combination with any of the previously indicated embodiments of the first aspect of the invention, the modified sequence of SD5 is characterized by comprising at least 2, 3, 4, 5, 6, 7, 8, 9, 10, 15, 20, 25, 30, 35, 40, 45 or 50 double mutations with respect to SEQ ID NO 2 selected from the list of mutations indicated in Table 6, and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In a second aspect, the present invention provides a composition comprising a modified sequence as defined in the first aspect.

In one embodiment of the second aspect of the invention, the composition comprises a modified sequence having a percentage of sequence identity of at least 85% with any of sequences SEQ ID NO 1 to 6; wherein preferably the administration of said modified sequence in a patient in need thereof reduces the succinate levels post-administration of said modified sequence in comparison to the succinate levels pre-administration of said modified sequence in a biological sample taken or isolated from said patient;
wherein said biological sample is preferably selected from blood, serum, plasma, urine, stool, semen, tears, mucus, sweat, milk, cerebrospinal fluid and/or saliva/buccal swabs.

In another embodiment of the second aspect of the invention, optionally in combination with any of the further embodiments of the second aspect of the invention, the composition comprises a modified sequence having a percentage of sequence identity of at least 85% with any of sequences SEQ ID NO 1 to 6 and is capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the second aspect of the invention, optionally in combination with any of the further embodiments of the second aspect of the invention, the composition comprises a modified sequence having a percentage of sequence identity of at least 85% with any of sequences SEQ ID NO 1 or 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the second aspect of the invention, optionally in combination with any of the further embodiments of the second aspect of the invention, the composition comprises a modified sequence of cis-aconitate decarboxylase (SD1, SEQ ID NO: 1) having a percentage of sequence identity of at least 85% with sequence SEQ ID NO 1, and preferably capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

The modified sequence of SEQ ID NO 1 is as defined in any of the embodiments of the first aspect of the invention.

In another embodiment of the second aspect of the invention, optionally in combination with any of the further embodiments of the second aspect of the invention, the composition comprises a modified sequence characterized by comprising at least one or more mutations with respect to SEQ ID NO 1, wherein the modified sequence is characterized by comprising the amino acid sequence represented by SEQ ID NO 1 with at least one substitution in the corresponding amino acid position of the modified amino acid sequence with respect to SEQ ID NO 1 selected from the list of Table 2 and preferably capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the second aspect of the invention, optionally in combination with any of the further embodiments of the second aspect of the invention, the composition comprises a modified sequence characterized by comprising at least two or more mutations with respect to SEQ ID NO 1, wherein the modified sequence is characterized by comprising the amino acid sequence represented by SEQ ID NO 1 with at least two substitutions in the corresponding amino acid position of the modified amino acid sequence with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the second aspect of the invention, optionally in combination with any of the further embodiments of the second aspect of the invention, the composition comprises a modified sequence characterized by comprising at least three or more mutations with respect to SEQ ID NO 1, wherein the modified sequence is characterized by comprising the amino acid sequence represented by SEQ ID NO 1 with at least three substitutions in the corresponding amino acid position of the modified amino acid sequence with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the second aspect of the invention, optionally in combination with any of the further embodiments of the second aspect of the invention, the composition comprises a modified sequence characterized by comprising at least four or more mutations with respect to SEQ ID NO 1, wherein the modified sequence is characterized by comprising the amino acid sequence represented by SEQ ID NO 1 with at least four substitutions in the corresponding amino acid position of the modified amino acid sequence with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the second aspect of the invention, optionally in combination with any of the further embodiments of the second aspect of the invention, the composition comprises a modified sequence characterized by comprising at least five or more mutations with respect to SEQ ID NO 1, wherein the modified sequence is characterized by comprising the amino acid sequence represented by SEQ ID NO 1 with at least five substitutions in the corresponding amino acid position of the modified amino acid sequence with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the second aspect of the invention, optionally in combination with any of the further embodiments of the second aspect of the invention, the composition comprises a modified sequence characterized by comprising at least all mutations with respect to SEQ ID NO 1, wherein the modified sequence is characterized by comprising the amino acid sequence represented by SEQ ID NO 1 with at least all substitutions in the corresponding amino acid position of the modified amino acid sequence with respect to SEQ ID NO 1 selected from the list of Table 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment of the second aspect of the invention, optionally in combination with any of the further embodiments of the second aspect of the invention, the composition comprises a modified sequence having a percentage of sequence identity of at least 85% with SEQ ID NO 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

The modified sequence of SEQ ID NO 2 is as defined in any of the embodiments of the first aspect of the invention.

Particularly, the composition comprises a modified sequence characterized by comprising at least one or more mutations with respect to SEQ ID NO 2, wherein the modified sequence is characterized by comprising the amino acid sequence represented by SEQ ID NO 2 with at least one substitution in the corresponding acid sequence represented by SEQ ID NO 2 with at least one substitution in the corresponding amino acid position of the modified amino acid sequence with respect to SEQ ID NO 2 selected from the list of Table 5, and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment, the composition comprises a modified sequence of SD5 characterized by comprising at least 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240 or 250 mutations with respect to SEQ ID NO 2 selected from the list of Table 5, and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In another embodiment, the composition comprises a modified sequence of SD5 characterized by comprising at least one double mutation with respect to SEQ ID NO 2, wherein the modified sequence is characterized by comprising the amino acid sequence represented by SEQ ID NO 2 with at least one double substitution in the corresponding amino acid positions of the modified amino acid sequence with respect to SEQ ID NO 2 selected from the list Table 6, and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction. In another embodiment, the composition comprises a modified sequence of SD5 characterized by comprising at least 2, 3, 4, 5, 6, 7, 8, 9, 10, 15, 20, 25, 30, 35, 40, 45 or 50 double mutations with respect to SEQ ID NO 2 selected from the list of Table 6, and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

In a third aspect, the present invention relates to a composition as defined in the first or second aspect of the invention for use in therapy.

The term "therapy" or "treatment", as used herein, refers to the attempted remediation of a health problem, usually following a diagnosis, or to prevention of the appearance of a health problem. As such, it is not necessarily a cure, i.e., a complete reversion of a disease. Said therapy may or may not be known to have a positive effect on a particular disease. This term includes both therapeutic treatment and prophylactic or preventive measures, in which the object is to prevent or stop (reduce) an undesired physiological change or disorder. For the purpose of this invention, beneficial or desired clinical results include, without limitation, relieving symptoms, reducing the spread of the disease, stabilizing pathological state (specifically not worsening), slowing down or stopping the progression of the disease, improving or mitigating the pathological state and remission (both partial and complete), both detectable and undetectable. It can also involve prolonging survival, disease free survival and symptom free survival, in comparison with the expected survival if treatment is not received. Those subjects needing treatment include those subjects already suffering the condition or disorder, as well as those with the tendency to suffer the condition or disorder or those in which the condition or disorder must be prevented.

The fourth aspect of the invention provides a composition comprising an enzyme as defined in the first or the second aspect of the invention, for use in a method for the prophylactic and/or therapeutic treatment of a disease associated with increased levels of succinate in a patient in need thereof.

A fifth aspect of the invention provides a method for the prophylactic and/or therapeutic treatment of a disease associated with increased levels of succinate in a patient, comprising the administration of a therapeutically effective amount of an enzyme as defined in the first or the second aspect of the invention to a subject in need thereof. Moreover, it pertains to the use of an enzyme as defined in the first or the second aspect in the manufacture of a medicament for the prophylactic and/or therapeutic treatment of a disease associated with increased levels of succinate in a patient in need thereof.

Beyond the capacity of reducing succinate levels shown *in vitro,* the inventors have demonstrated that SD1 and SD5 enzymes were effective in reducing succinate levels *in vivo.* Cis-aconitate (SD1) enzyme reduced succinate levels in a murine model of obesity and type 2 diabetes, as shown by the lower recovery of circulating succinate levels 6 hours after fasting (Fig. 16), ina NAFLD model (Fig. 18) and in a model of DSS-induced colitis (Fig. 20). SD1 ameliorated glucose tolerance in NAFLD (Fig. 17) and reduced disease activity in moderate acute colitis (Fig. 19) and severe relapse colitis (Fig. 22). Both SD1 and SD5 improved mucosal healing and decrease the colon weight-to-length ratio and epithelium height in DSS-induced colitis (Fig. 21). Thus, the inventors have identified the *in vitro* and *in vivo* use of SD1 and SD5 and their variants to reduce the levels of succinate.

In one embodiment of the fifth aspect of the invention, the composition comprises an enzyme selected from the list consisting of cis-aconitate decarboxylase (SD1) of SEQ ID NO 1, ferulic acid decarboxylase 1 (SD5) of SEQ ID NO 2, or any of sequences SEQ ID NO 3 (SD2), SEQ ID NO 4 (SD3), SEQ ID NO 5 (SD4) or SEQ ID NO 6 (SD6), or a modified sequence having a percentage of sequence identity of at least 85% with any of sequences SEQ ID NO 1 to 6 and capable of reducing the circulating succinate levels in a patient in need thereof, for use in a method for the prophylactic and/or therapeutic treatment of a disease associated with increased levels of succinate in a patient in need thereof.

In another embodiment of the fifth aspect of the invention, the enzyme is cis-aconitate decarboxylase (SD1) of SEQ ID NO 1, or a modified sequence having a percentage of sequence identity of at least 85% with SEQ ID NO 1 and capable of reducing the circulating succinate levels in a patient in need thereof.

The modified sequence of SEQ ID NO 1 is as defined in any embodiment of the first or the second aspect.

In another embodiment of the fifth aspect of the invention, the enzyme is cis-aconitate decarboxylase (SD1) of SEQ ID NO 1, or a modified sequence thereof characterized by comprising at least one or more mutations with respect to SEQ ID NO 1, wherein the modified sequence is characterized by comprising the amino acid sequence represented by SEQ ID NO 1 with at least one or more substitutions in the corresponding amino acid positions of the modified amino acid sequence with respect to SEQ ID NO 1 selected from the list of Table 2 and capable of reducing the circulating succinate levels in a patient in need thereof. In another embodiment, the enzyme is cis-aconitate decarboxylase (SD1) of SEQ ID NO 1, or a modified sequence thereof characterized by comprising at least two or more mutations with respect to SEQ ID NO 1, wherein the modified sequence is characterized by comprising the amino acid sequence represented by SEQ ID NO 1 with at least two or more substitutions in the corresponding amino acid positions of the modified amino acid sequence with respect to SEQ ID NO 1 selected from the list of Table 2 and capable of reducing the circulating succinate levels in a patient in need thereof. In another embodiment, the enzyme is cis-aconitate decarboxylase (SD1) of SEQ ID NO 1, or a modified sequence thereof characterized by comprising at least three or more mutations with respect to SEQ ID NO 1, wherein the modified sequence is characterized by comprising the amino acid sequence represented by SEQ ID NO 1 with at least three or more substitutions in the corresponding amino acid positions of the modified amino acid sequence with respect to SEQ ID NO 1 selected from the list of Table 2 and capable of reducing the circulating succinate levels in a patient in need thereof.

In another embodiment of the fifth aspect of the invention, the enzyme is cis-aconitate decarboxylase (SD1) of SEQ ID NO 1, or a modified sequence thereof characterized by comprising at least four or more mutations with respect to SEQ ID NO 1, wherein the modified sequence is characterized by comprising the amino acid sequence represented by SEQ ID NO 1 with at least four or more substitutions in the corresponding amino acid positions of the modified amino acid sequence with respect to SEQ ID NO 1 selected from the list of Table 2 and capable of reducing the circulating succinate levels in a patient in need thereof.

In another embodiment of the fifth aspect of the invention, the enzyme is cis-aconitate decarboxylase (SD1) of SEQ ID NO 1, or a modified sequence thereof characterized by comprising at least five or more mutations with respect to SEQ ID NO 1, wherein the modified sequence is characterized by comprising the amino acid sequence represented by SEQ ID NO 1 with at least five or more substitutions in the corresponding amino acid positions of the modified amino acid sequence with respect to SEQ ID NO 1 selected from the list of Table 2 and capable of reducing the circulating succinate levels in a patient in need thereof.

In another embodiment of the fifth aspect of the invention, the enzyme is cis-aconitate decarboxylase (SD1) of SEQ ID NO 1, or a modified sequence thereof characterized by comprising at least all mutations with respect to SEQ ID NO 1, wherein the modified sequence is characterized by comprising the amino acid sequence represented by SEQ ID NO 1 with at least all substitutions in the corresponding amino acid positions of the modified amino acid sequence with respect to SEQ ID NO 1 selected from the list of Table 2 and capable of reducing the circulating succinate levels in a patient in need thereof.

In another embodiment of the fifth aspect of the invention, the enzyme is ferulic acid decarboxylase 1 (SD5) of SEQ ID NO 2 or a modified sequence having a percentage of sequence identity of at least 85% with SEQ ID NO 2 and capable of reducing the circulating succinate levels in a patient in need thereof.

The modified sequence of SEQ ID NO 2 is as defined in any embodiment of the first or the second aspect.

In another embodiment of the fifth aspect of the invention, the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least one or more substitutions in the corresponding amino acid position of the modified amino acid sequence with respect to SEQ ID NO 2selected from the list of Table 5 and capable of reducing the circulating succinate levels in a patient in need thereof. In another embodiment, the enzyme is ferulic acid decarboxylase 1 (SD5) of SEQ ID NO 2, or a modified sequence thereof characterized by comprising at least 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240 or 250 mutations with respect to SEQ ID NO 2 selected from the list of Table 5 and capable of reducing the circulating succinate levels in a patient in need thereof.

In another embodiment of the fifth aspect of the invention, the enzyme is ferulic acid decarboxylase 1 (SD5) of SEQ ID NO 2, or a modified sequence thereof characterized by comprising at least one double mutation with respect to SEQ ID NO 2, wherein the modified sequence is characterized by comprising the amino acid sequence represented by SEQ ID NO 2 with at least one double substitution in the corresponding amino acid positions of the modified amino acid sequence with respect to SEQ ID NO 2 selected from the list of Table 6 and capable of reducing the circulating succinate levels in a patient in need thereof. In another embodiment, the enzyme is ferulic acid decarboxylase 1 (SD5) of SEQ ID NO 2, or a modified sequence thereof characterized by comprising at least 2, 3, 4, 5, 6, 7, 8, 9, 10, 15, 20, 25, 30, 35, 40, 45 or 50 double mutations with respect to SEQ ID NO 2 selected from the list of Table 6 and capable of reducing the circulating succinate levels in a patient in need thereof.

Diseases associated with increased levels of succinate are, without being limited to, hypertension, ischemia, cancer, obesity, type 2 diabetes mellitus, non-alcoholic fatty liver disease (NAFLD), non-alcoholic steatohepatitis (NASH), inflammatory bowel disease (IBD), including Cronh's disease and ulcerative colitis. Preferably, the disease is selected from the list consisting of obesity, type 2 diabetes mellitus, non-alcoholic fatty liver disease (NAFLD) and ulcerative colitis.

Inflammatory bowel disease (IBD) is a continuum of diseases that comprise Crohn's disease (CD), ulcerative colitis (UC), and indeterminate colitis. UC is characterized with inflammation of the colonic mucosa and encompasses a wide spectrum of disease presentations and clinical courses. Common features of UC are diarrhea and bleeding. The course of UC is relapsing and remitting. The majority of patients have a mild to moderate disease, but around 15-25% of patients with UC will experience at least one episode of severe flare of their disease and 10-20% will present with acute severe ulcerative colitis (ASUC) at diagnosis (Rosiou K, Selinger CP. Acute severe ulcerative colitis: management advice for internal medicine and emergency physicians. Intern Emerg Med. 2021 Sep;16(6):1433-1442. doi: 10.1007/s11739-021-02704-0). CD may involve any aspect of the gastrointestinal (GI) tract, from mouth to anus. Disease severity and location dictate the associated signs and symptoms, which leads to a wide spectrum of clinical presentations. Classic characteristics of CD comprise abdominal pain, watery diarrhea and weight loss (Flynn S, Eisenstein S. Inflammatory Bowel Disease Presentation and Diagnosis. Surg Clin North Am. 2019 Dec;99(6):1051-1062. doi: 10.1016/j.suc.2019.08.001).

In a particular embodiment, the composition comprises an enzyme as defined in the first aspect or second aspect of the invention for use in a method of therapy for reducing the intestinal succinate.

In another embodiment of the fifth aspect of the invention, the composition comprising an enzyme as defined in the first or the second aspect of the invention is administered in combination with at least one or more treatments.

Therapeutic approaches for IBD include pharmacological interventions such as aminosalicylates (e.g., sulfasalazine and other 5-aminosalicylic acid (5-ASA)), corticosteroids, immunomodulators (e.g., thiopurines, methotrexate, calcineurin inhibitors and Janus Kinase inhibitors), biologics (e.g., pro-inflammatory cytokine inhibitors and integrin antagonists), surgical treatment, novel therapies including apheresis therapy, improved intestinal microecology (e.g., antibiotics, probiotics, prebiotics, synbiotics and postbiotics, fecal microbiota transplant), cell therapy and exosome therapy (Cai Z, Wang S, Li J. Treatment of Inflammatory Bowel Disease: A Comprehensive Review. Front Med (Lausanne). 2021 Dec 20;8:765474. doi: 10.3389/fmed.2021.765474).

As in IBD, treatments for CD include corticosteroids, 5-ASA, antibiotics (e.g. metronidazole and others), thiopurines, methotrexate, anti-TNF biologics (e.g. infliximab, adalimumab, certolizumab pegol, vedolizumab, ustekinumab) and biosimilars (Kumar A, Cole A, Segal J, Smith P, Limdi JK. A review of the therapeutic management of Crohn's disease. Therap Adv Gastroenterol. 2022 Feb 17;15:17562848221078456. doi: 10.1177/17562848221078456.)

Suitable treatments for UC management include corticosteroids (such as cortisone, prednisolone, hydrocortisone, budesonide multimatrix (MMX), beclomethasone dipropionate), sulfasalazine and 5-aminosalicylic acid (5-ASA), including topical steroids and 5-ASA, thiopurines (like azathioprine), cyclosporin, anti-tumor necrosis factors (such as infliximab, adalimumab, golimumab), anti-integrin agents (including vedolizumab), anti-interleukin agents (such as ustekinumab), Janus kinase inhibitors (like tofacitinib, upadacitinib, filgotinib) and/or sphingosine-1-phospate receptor modulators (namely ozanimod). Novel therapeutic agents which under development comprise abrilumab, AJM300, mirikizumab, risankizumab, guselkumab, etrasimod, cobitolimod and/or faecal microbiota transplant (Aslam N, Lo SW, Sikafi R, Barnes T, Segal J, Smith PJ, Limdi JK. A review of the therapeutic management of ulcerative colitis. Therap Adv Gastroenterol. 2022 Nov 29;15:17562848221138160. doi: 10.1177/17562848221138160.)

In another embodiment of the fifth aspect of the invention, the composition comprising an enzyme as defined in the first or the second aspects of the invention is administered in combination with at least one or more treatments suitable for IBD management, including UC and CD.

Obesity is commonly assessed by the BMI (obese BMI≥ 30 kg/m²). Initial treatment recommendations for obesity include lifestyle interventions (such as healthy nutrition, increased physical activity and behavioral modifications). Since weight loss is usually moderate, bariatric surgery and drugs are also used (Atlas SJ, Kim K, Nhan E, Touchette DR, Moradi A, Agboola F, Rind DM, Beaudoin FL, Pearson SD. Medications for obesity management: Effectiveness and value. J Manag Care Spec Pharm. 2023 May;29(5):569-575. doi: 10.18553/jmcp.2023.29.5.569). Anti-obesity pharmacological approaches include sympathicomimetics (such as diethylpropion/afepramone, phenmetrazine, phendimetrazine, phenylpropanolamine, fenfluramine and dexfenfluramine, cathine (nor-pseudoephedrine), sibutramine, phentermine, CB1 receptor blockers (like rimonabant), pancreatic lipase inhibitor (such as orlistat), 5-HT_{2C} serotonin agonists (lorcaserin), sympathomimetics/anticonvulsants (phentermine/topiramate ER), opioid receptor antagonist/dopamine and noradrenaline reuptake inhibitor (namely naltrexone SR/bupropion SR), GLP1R agonists (liraglutide, semaglutide). Other weight loss drugs that are under investigation are GLP1/glucagon dual agonists (such as cotadutide, BI 456906, efinopegdutide, OXM), GIP/GLP1 dual agonists (like tirzepatide, GIP/GLP peptide I, GIP/GLP peptide II, NN9709), GIP/GLP1/glucagon tri-agonists (e.g. HM15211, GGG tri-agonist, NN9423), GIPR agonists (e.g. GIPR agonist long acting, ZP 6590), GLP1R agonists (e.g. efpeglenatide, rybelsus, danuglipron, GLPR-NPA, PF-07081532), glucagon analogue (e.g. HM15136), leptin sensitizers (e.g. withaferin A, celastrol, leptin/amylin), Y2R agonists (e.g. PYY analogue, NN9748, NNC0165-1875+semaglutide), amylin/calcitonin dual agonists (e.g. KBP-098, KBP-042, davalintide), amylin analogues (e.g. cagrilintide, ZP8396), drugs targeting the fhrelin pathway (e.g. CYT009-GhrQb), Nox-B11, azp-531), mitochondrial uncouplers (such as BAM15), other appetite suppressants (e.g. GDF15, LY-3463251, JNJ-9090/CIN-109) (Müller TD, Blüher M, Tschöp MH, DiMarchi RD. Anti-obesity drug discovery: advances and challenges. Nat Rev Drug Discov. 2022 Mar;21(3):201-223. doi: 10.1038/s41573-021-00337-8.).

In another embodiment of the fifth aspect of the invention, the composition comprising an enzyme as defined in the first or the second aspect of the invention is administered in combination with at least one or more treatments against obesity.

Type 2 diabetes mellitus (T2DM) is characterized by hyperglycaemia with a varying degree of insulin resistance. T2DM medications include metformin, sulfonylureas (e.g. glibenclamide, glicazide, glipizide), meglitinides (e.g. nateglinide, repaglinide), dipeptidyl peptidase 4 inhibitors (e.g. alogliptin, linagliptin, saxagliptin, sitagliptin vildagliptin), sodium-glucose cotransporter2 inhibitors (e.g. canagliflozin, dapagliflozin, empagliflozin, etugliflozin), GLP1 receptor agonists (e.g. dulaglutide, exenatide, liraglutide, lixisenatide, semaglutide), thiazolidinediones (e.g. pioglitazone), α-glucosidase inhibitors (e.g. acarbose), insulin (including ultra-rapid-acting, Fiasp; rapid-acting, Aspart, glulisine or lispro; short-acting, Actrapid, Humulin S or Insuman rapid; intermediate: insulatard or Humulin I; long-acting, degludec, detemir or glargine; biphasic (pre-mixed), Humalog, Humulin M3 or Novomix) and/or other glucose-lowering agents (Bellary S, Kyrou I, Brown JE, Bailey CJ. Type 2 diabetes mellitus in older adults: clinical considerations and management. Nat Rev Endocrinol. 2021 Sep;17(9):534-548. doi: 10.1038/s41574-021-00512-2).

In another embodiment of the fifth aspect of the invention, the composition comprising an enzyme as defined in the first or the second aspect of the invention is administered in combination with at least one or more treatments for T2DM.

Non-alcoholic fatty liver disease (NAFLD, also named as metabolic-associated fatty liver disease, MAFLD) is a spectrum of disease characterized by hepatic steatosis (when secondary causes of hepatic fat accumulation cannot be identified, such as excessive alcohol consumption). NAFLD comprises non-alcoholic fatty (NAFL) to non-alcoholic steatohepatitis (NASH). NAFLD may progress to fibrosis and cirrhosis. Treatment for NAFLD can include a combination of lifestyle modifications, increasing physical activity and smoking/alcohol cessation, healthy diet (reduction of caloric intake and high-glycaemic index foods, increased consumption of monounsaturated fatty acids, omega-3 fatty acids, fibers, and specific protein sources such as fish and poultry, and weight loss. Some medications for NAFLD that have shown promising outcomes are pioglitazone and GLP-1 receptor agonists. Bariatric and metabolic surgery are also therapeutic options for NAFLD patients.

In another embodiment of the fifth aspect of the invention, the composition comprising an enzyme as defined in the first or the second aspect of the invention is administered in combination with at least one or more treatments for NAFLD.

### Route of administration

The composition comprising an enzyme as defined in the first or the second aspect for use according to the fourth of fifth aspect is typically formulated as a pharmaceutical composition to be compatible with its intended route of administration. Methods to accomplish the administration are known to those of ordinary skill in the art. This includes, for example, injection or infusion, by parenteral routes such as intravascular (e.g., intravenous or intraarterial), subcutaneous, intramuscular, intraperitoneal, intraventricular, intraepidural, or others as well as oral, sublingual, nasal, ophthalmic, intrathecal, intracranial, rectal, transdermal or topical. Sustained release administration is also specifically contemplated, e.g., as depot injections or erodible implants. Localized delivery is particularly contemplated, e.g., as delivery via a catheter to one or more arteries or a vessel supplying a localized site of interest. Preferably, the composition of the invention is administered through the oral or rectal route. More preferably, the composition of the invention is administered through the oral route.

In one embodiment, the composition of the invention is formulated in order to reach the colon.

Oral dosage forms can comprise tablets, capsules, syrups, suspensions, solutions and chewable tablets. Preferably, the composition of the invention is formulated as a gastro-resistant capsule or a capsule suitable for colonic delivery.

Rectal drug delivery can be liquid dosage forms (e.g., enemas), solid dosage forms (e.g., suppositories, capsules and tablets), and semi-solid dosage forms (e.g., gels, foams, and creams) (Hua S. Physiological and Pharmaceutical Considerations for Rectal Drug Formulations. Front Pharmacol. 2019 Oct 16;10:1196. doi: 10.3389/fphar.2019.01196).

In the context of the present invention, a "gut-restricted" or "intestinally-restricted" drug or composition refers to a drug or composition that remains confined to the gastrointestinal tract upon oral uptake because of their limited passive permeability, increasing the local concentration at the site of action resulting in a minimized exposure of the rest of the body (Dorel R, Wong AR, Crawford JJ. Trust Your Gut: Strategies and Tactics for Intestinally Restricted Drugs. ACS Med Chem Lett. 2023 Feb 22;14(3):233-243. doi: 10.1021/acsmedchemlett.3c00001). More preferably, the composition is formulated for an oral, gut-restricted and colonic delivery.

### Administration schedule and dosage

The composition comprising an enzyme as defined in the first or the second aspect for use according to the fourth or fifth aspect can be administered a single time. It may also be administered regularly throughout the course of the method of treatment, for example, one, two, three, four, or more times a day, every other day, weekly, bi-weekly, every three weeks or monthly.

The dosage to be administered can depend on multiple factors, including the type and severity of the disease and/or on the characteristics of the subject, such as general health, age, sex, body weight and tolerance to drugs and should be adjusted, as needed, according to individual need and professional judgment. The dosage may also vary depending upon factors, such as route of administration, treatment regime, target site, or other therapies administered. The skilled artisan will be able to determine appropriate doses depending on these and other factors. A prophylactic or therapeutically effective amount may include, but is not limited to, dosage ranges of about 0.1 mg/kg to about 100 mg/kg of body weight, preferably about 1 mg/kg to about 20 mg/kg, more preferably, about 2 mg/kg to about 15 mg/kg.

### Pharmaceutical composition

The present invention also provides a pharmaceutical composition comprising an enzyme as as defined in the first or the second aspect and a pharmaceutically acceptable excipient, preferably for use according to the fourth or fifth aspect.

Pharmaceutically acceptable excipients include, but are not limited to a carrier or diluent, such as a gum, a starch (e.g. corn starch, pregeletanized starch), a sugar (e.g. lactose, mannitol, sucrose, dextrose), a cellulosic material (e.g. microcrystaline cellulose), an acrylate (e.g. polymethylacrylate), calcium carbonate, magnesium oxide, talc, or mixtures thereof; a binder (e.g. acacia, cornstarch, gelatin, carbomer, ethyl cellulose, guar gum, hydroxypropyl cellulose, hydroxypropyl methyl cellulose, povidone); a disintegrating agent (e.g. cornstarch, potato starch, alginic acid, silicon dioxide, croscarmelose sodium, crospovidone, guar gum, sodium starch glycolate), a buffer (e.g. Tris-HCl, acetate, phosphate, bicarbonate) of various pH and ionic strength; and additive such as albumin or gelatin to prevent absorption to surfaces; a detergent (e.g. Tween 20, Tween 80, Pluronic F68, bile acid salts); a protease inhibitor; a surfactant (e.g. sodium lauryl sulfate); a permeation enhancer; a solubilizing agent (e.g. glycerol, polyethylene glycerol); an antioxidants (e.g. ascorbic acid, sodium metabisulfite, butylated hydroxyanisole); a stabilizer (e.g. hydroxypropyl cellulose, hydroxypropylmethyl cellulose); a viscosity increasing agent (e.g. carbomer, colloidal silicon dioxide, ethyl cellulose, guar gum); a sweetener (e.g. aspartame, citric acid); a preservative (e.g. thimerosal, benzyl alcohol, parabens); a lubricant (e.g. stearic acid, magnesium stearate, polyethylene glycol, sodium lauryl sulfate); a flow-aid (e.g. colloidal silicon dioxide), a plasticizer (e.g. diethyl phthalate, triethyl citrate); an emulsifier (e.g. carbomer, hydroxypropyl cellulose, sodium lauryl sulfate); a polymer coating (e.g. poloxamers or poloxamines); a coating and film forming agent (e.g. ethyl cellulose, acrylates, polymethacrylates); a pharmaceutically acceptable carrier for liquid formulations, such as an aqueous (water, alcoholic/aqueous solution, emulsion or suspension, including saline and buffered media) or non-aqueous (e.g., propylene glycol, polyethylene glycol, and injectable organic esters, such as ethyl oleate) solution, suspension, emulsion or oil; and a parenteral vehicle (e.g., for subcutaneous, intravenous, intraarterial, or intramuscular injection), including but not limited to, water, oils, saline solution, Ringer's dextrose, aqueous dextrose and other sugar solutions. A pharmaceutically acceptable excipient also includes excipients for nanoencapsulation purposes, such as a cationic polyelectrolyte (e.g. gelatin and an anionic polyelectrolyte (e.g. arabic gum).

It is contemplated that any embodiment discussed in this specification can be implemented with respect to any medical use, pharmaceutical composition, method of treatment, method of manufacturing a medicament and combination therapies of the invention, and vice versa. It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications are herein incorporated by reference to the same extent as if each individual publication or patent application was specifically and individually indicated to be incorporated by reference.

The following examples serve to illustrate the present invention and should not be construed as limiting the scope thereof.

### EXAMPLES

### Materials and methods

### Succinate and fumarate docking and molecular dynamics for SD1

First, cis-aconitate was docked in 3 active site conformations corresponding to the active site found in AlphaFold model and the 2 conformations (chain A and B) obtained after refinement of the dimer model with Rosetta. Then, cis-aconitate binding mode was equilibrated in 3 MD simulations for 3ns. On the active sites equilibrated with cis-aconitate, succinate and fumarate were docked. Binding energies were predicted with Vina 1.2 and best stabilization of succinate and fumarate was obtained in the active sites of the AlphaFold model after equilibration with the natural substrate cis-aconitate. The next step was to select the lowest energy binding modes that span different clusters and use the best behaving enzyme conformation to equilibrate and refine those binding modes during 3ns MDs simulations. Two different MD simulations were used per ligand to enhance sampling and samled a total of 4 (2 MD replicas x 2 active sites per dimer) low energy binding modes per ligand.

### Succinate and fumarate docking and molecular dynamics for SD5

SD5 dimer was built starting from the crystallographic structure 4S13 and missing residues were built using information available in each monomer and in the homologous structure 4IWS. SD5's active site was modelled using the PDB structure 7ABN to guide the modelling of prFMN cofactor. The dimer was then refined using the FastRelax protocol from Rosetta. Then, the active site geometry was refined by docking the natural substrate, ferulic acid, for subsequent equilibration of the active site through MD simulations. Ferulic acid was then docked in all active sites (chain A and C) of all SD5 dimer models. The binding mode predictions were then clustered according to the geomery to identify diverse binding modes. Ferulic acid binding modes were equilibrated in 4MD simulations for 3ns. The equilibration of ferulic acid binding modes using MD simulations was used to refine the active site environment and predict succinate and fumarate binding modes.

Succinate and fumarate were docked with Vina 1.2 and the binding modes were clustered according to geometry. The binding modes of succinate and fumarate were refined during 3ns MDs simulations.

### Semi-rational mutagenesis and variant screening

Based on docking, molecular dynamics and predicted binding modes, selected aminoacidic positions were chosen and proposed for mutations. New genes were synthesized, cloned into pET22 or pBAD vectors, produced at small scale and tested fur succinate activity

### Succinate-depleting enzymatic activity measurement

To measure succinate depleting activity decarboxylases SD1 and SD5, reactions with succinate as substrate were performed. In order to do so, **in vitro** reactions were prepared as follows:
- 10 uM of enzyme
- 200 mM NaHPO4- buffer
- 200 uM succinic acid

Besides, a control sample was carried out without enzyme at the same buffer and succinic acid concentration. Once reactions were prepared, they were incubated for 24h at 37°C. After it, reactions were stopped with a 20-minute incubation at 80°C. Remaining succinate was measured with a bioluminescent-based enzymatic Succinate-Glo^{™} kit (V7990, Promega, Madison, Wisconsin, USA) using the Varioskan Lux, a luminescence Reader (VL0000D0, Thermo Scientific, Waltham, Massachusetts, USA).

### Succinate, fumarate and malate measurement by LC-MS/MS

To assess the activity of the enzymes, we conducted two kind of reactions: 24-h reactions with succinate as initial substrate in which we aimed to measure succinate reduction and fumarate increase; and reactions using C¹³-labeled succinate as the initial substrate, measuring the products at different time points (0, 2, 6, and 24 hours) to study the kinetics of the reaction. The remaining succinate and produced fumarate and malalte in these reactions were measured by liquid chromatography with triple-quadruple mass spectrometry (LC-QqQ MS). Metabolites were extracted by mixing 150 µL of methanol with 150 µL of sample, followed by vortexing. The mixture was incubated at 4°C for 30 minutes while shaking. Then, samples were diluted again with 150 µL of methanol reaching a final concentration of 25% Methanol. Targeted analysis of succinate, fumarate and malate performed using an ultra-high-performance LC system coupled to a 6490 QqQ mass spectrometer (Agilent Technologies, Palo Alto, CA) with iFunnel technology in negative electrospray ionization (ESI). Metabolites were retained and separated by hydrophilic interactions using an InfinityLab Poroshell 120 HILIC-Z, 2.1 × 100 mm, 2.7 µm (PEEK lined) (Agilent Technologies) column. Mobile phase A was 50 mM ammonium acetate and 5 µM medronic acid in water, and mobile phase B was acetonitrile. Five microliters of the extracts were injected into the LC-MS/MS system. Peak area was manually integrated using Mass Hunter Qualitative software (Agilent Technologies).

### Succinate measurement in plasma, feces and cell culture media samples

Plasma and fecal samples from 16-h fasted mice and culture media from *in vitro* experiments were filtered (10 kDa) and succinate levels were determined using a fluorometric assay (EnzyChrom Succinate Assay Kit; BioAssay Systems, Hayward, CA).

### Cell-based assays

The human liver cancer HepG2 and colonic cancer HT29 cell lines were obtained from the ATCC (Rockville, MD, USA). HT29 cells were cultured in Dulbecco's Modified Eagle's Medium (DMEM) supplemented with 10% FBS and 1% antibiotics/antimycotics at a density of 20,000 cells/cm². HepG2 cells were propagated in DMEM/F12 supplemented with 1% L-glutamine (Sigma-Aldrich), 10% FBS, 1% antibiotics/antimycotics solution and 2% HEPES (HyClone, Logan, UT, USA) at a density of 40,000 cells/cm2. All cells were cultured in a humidified incubator at 37 °C with 5% CO2. Experiments using conditioned medium (CM) were performed using 24 h cultures. HT29 cells were treated with several concentrations of succinate and/or enzymes (SD5-His and SD1-SUMO) and, after 24h, media was collected for succinate determination and RNA was extracted for gene expression analysis. HepG2 cells were treated with insulin and/or succinate and/or the enzyme SD5-His. After 24h, cells were lysed to extract the protein content to perform western blot analysis.

### Western Blot

Cells were lysed and homogenized in M-PER buffer (Thermo Scientific) containing Halt Protease and Phosphatase Inhibitor Cocktails (Thermo Scientific). Protein concentration was determined using the BCA Protein Assay Kit (Thermo Scientific). Equal amounts of total protein were separated via SDS-polyacrylamide gel electrophoresis, transferred to Immobilon-P PVDF membranes (Merck Millipore, Burlington, MA), and blocked. Immunoblot analysis was performed with a polyclonal antibody against phospho(p)-AKT (Ser473) (Cell Signaling Technology, Danvers, MA) and a monoclonal antibody against β-ACTIN (Sigma-Aldrich) as loading control. Membranes were incubated with the respective HRP-conjugated secondary antibodies and visualized using the Immobilon ECL Ultra Western HRP Substrate (Merck Millipore). Band intensity was captured with the iBright CL1000 Imaging System and analyzed using iBright Analysis Software (Invitrogen, Waltham, MA).

### Gene Expression Assays

Total RNA was extracted from tissues or cells using TRIzol Reagent (Invitrogen, Carlsbad, CA). cDNA was synthesized using the High-Capacity cDNA Reverse Transcription Kit (Applied Biosystems, Carlsbad, CA), and quantitative real-time PCR was performed on a 7900HT Fast Real-Time PCR System using the TaqMan Gene Expression Assay (IL6: Mm00446190_m1, TNF: Mm00446190_m1, NPLR3: Hs00429221_m1, B2m: Mm00437762_m1, RNA18S5: Hs03928989_g1; Applied Biosystems). B2m and RNA18S5 served as housekeeping genes for mice tissue samples and human cell culture samples, respectively. Relative expression levels were calculated using the comparative Ct method (2-ΔΔCt).

### DSS-induced colitis mice models

### A) Moderate acute colitis

Thirty-two C57BL/6J mice (50% female, 50% male) obtained at Charles Rivers Laboratory (Barcelona, Spain). They were administrated 2% DSS (MFCD00081551, MP Biomedicals, Santa Ana, California, USA) via oral for 6 days to induce acute colitis. Then, mice were distributed into 3 groups for 7 days of treatment. One was treated with an empty vector, another with SD1 enzyme 7mg/kg and the last one with SD5 enzyme 7mg/kg and all of them were administrated via intracolonic canulation after 2 hours of fasting.

Body weight and food ingested were weighed every other day. Moreover, Score Stool Consistency and Score Stool Bleeding were evaluated once every other day to calculate the Disease Activity Index (DAI) (Formula 1). At 7-day, mice were anesthetized with isoflurane and killed by cardiac exsanguination. In all cases, faecal content, blood, plasma, caecum and colon samples were obtained after sacrifice and immediately were frozen and stored at -80°C. Mice maintenance and their samples extraction were supplied by qualified members of the group.

### B) Severe relapse colitis

Sixteen male mice C57BL/6J mice obtained at Charles Rivers Laboratory (Barcelona, Spain). They were administrated 3.5% DSS (MFCD00081551, MP Biomedicals, Santa Ana, California, USA) via oral for 7 days to induce acute colitis, followed by a 9-day wash-out period. Since day 11, treatments with enzyme SD1 7mg/kg, Tofacitinib 1mg/kg and empty vector as vehicle were administered daily, after 2h of fasting. At day 16, a colitis relapse was induced with DSS 1% for four days. After that, a second wash-out of two days was performed. DAI was registered and samples were obtained equally as in the moderate acute DSS model.

### NAFLD Mice Model

Forty-eight male C57BL/6J mice from Charles Rivers Laboratory (Barcelona, Spain) were fed a choline-deficient high-fat diet (60% fat) with 0.1% methionine (CDA-HFD) (A06071302, Research Diets, New Brunswick, USA) for 7 weeks. Following overnight fasting, a glucose tolerance test (GTT) was conducted by injecting 2 g/kg of glucose and measuring blood glucose at 0, 15, 30, 60, and 120 minutes using a glucometer (Accu-Check Instant, Roche, Basel, Switzerland). Mice were then divided into four groups for 2 weeks of treatment. The vehicle group received an empty vector, while two groups were treated with SD1 and SD5 enzymes at high and low doses (7 or 2 mg/kg) via intraperitoneal injection. The final group received oral pioglitazone at 10 mg/kg (MFCD00865504, Sigma-Aldrich, St. Louis, Missouri, USA) resuspended in 10% condensed milk. After 9 weeks, another GTT was performed following overnight fasting. Mice were anesthetized with isoflurane and euthanized by cardiac exsanguination. Plasma was obtained from cardiac blood, and livers were isolated, sectioned into two pieces, with one frozen and stored at -80°C and the other fixed in 4% paraformaldehyde.

### Histology

Histological analyses of murine colon and liver samples were performed on tissues fixed in paraformaldehyde (PFA) and stained with hematoxylin and eosin (H&E) at the Pathological Anatomy Unit of the Institut Català de la Salut de les Terres de I'Ebre. Histological images were viewed using SilderViewer (2.7.0.191696, 3DHISTECH, Budapest, Hungary). In the liver histology analysis of NAFLD mice, imaged software (RRID: SDR_003070, National Institutes of Health, USA) was used to select and quantify the area, number, and size of fat vacuolations, evaluating the degree of damage. H&E staining of liver histology was used to analyze the development of hepatic steatosis, indicated by abnormal fat accumulation through lipid droplets (LDs) in hepatocytes, a key feature of NAFLD. NAFLD can range from simple steatosis to more severe steatohepatitis (MASH), where hepatocyte ballooning is the most specific indicator.

In order to analyse colon histology of UC mice, the histological assessment of Geboes Score (OGS) was used. This scoring system includes five grades: Grade 0 indicates no inflammatory activity, with subgrades ranging from 0.0 (no abnormalities), 0.1 (presence of architectural changes), to 0.2 (presence of architectural changes and chronic mononuclear infiltrate). Grade 1 assesses basal plasma cells, from 1.0 (no increase), 1.1 (mild increase), to 1.2 (marked increase). Grade 2A evaluates eosinophils in the lamina propria, from 2A.0 (no increase), 2A.1 (mild increase), to 2A.2 (marked increase). Grade 2B considers neutrophils in the lamina propria, from 2B.0 (no increase), 2B.1 (mild increase), to 2B.2 (marked increase). Grade 3 examines neutrophils in the epithelium, from 3.0 (none), 3.1 (less than 50% crypts involved), to 3.2 (more than 50% crypts involved). Finally, Grade 4 assesses epithelial injury in the crypt and surface epithelium, from 4.0 (none), 4.1 (marked attenuation), 4.2 (probable crypt destruction and probable erosions), 4.3 (unequivocal crypt destruction and unequivocal erosions), to 4.4 (ulcer or granulation tissue). [48]. Moreover, imaged was also used to measure the length of the epithelium and evaluated the degree of histologic damage.

### ITEMS OF THE INVENTION

*1. In vitro* use of a decarboxylase enzyme pertaining to carboxy-lyase EC 4.1.1. subclass for carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.
2. The use according to item 1, wherein the enzyme is selected from the list consisting of cis-aconitate decarboxylase (SD1) of SEQ ID NO 1, ferulic acid decarboxylase 1 (SD5) of SEQ ID NO 2, or any of sequences SEQ ID NO 3 (SD2), SEQ ID NO 4 (SD3), SEQ ID NO 5 (SD4) or SEQ ID NO 6 (SD6), or a modified sequence having a percentage of sequence identity of at least 85% with any of sequences SEQ ID NO 1 to 6 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.
3. The use according to item 1, wherein the enzyme is selected from the list consisting of cis-aconitate decarboxylase (SD1) of SEQ ID NO 1 and ferulic acid decarboxylase 1 (SD5) of SEQ ID NO 2, or a modified sequence having a percentage of sequence identity of at least 85% with any of sequences SEQ ID NO 1 or 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.
4. The use according to item 3, wherein the enzyme is cis-aconitate decarboxylase (SD1) of SEQ ID NO 1, or a modified sequence having a percentage of sequence identity of at least 85% with SEQ ID NO 1 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.
5. The use according to item 4, wherein the enzyme is cis-aconitate decarboxylase (SD1) of SEQ ID NO 1, or a modified sequence thereof characterized by comprising at least one or more mutations with respect to SEQ ID NO 1, wherein the modified sequence is characterized by comprising the amino acid sequence represented by SEQ ID NO 1 with at least one substitution in the corresponding amino acid position of the modified amino acid sequence with respect to SEQ ID NO 1 selected from the list of :

| Amino acid of SEQ ID NO 1 | Position in SEQ ID NO 1 | Amino acid of the modified sequence |
|---|---|---|
| L | 110 | W |
| H | 168 | A |
| H | 168 | K |
| K | 217 | A |
| K | 217 | Q |
| K | 288 | A |
| K | 288 | Q |
| G | 294 | A |
| H | 336 | A |
| H | 336 | K |

6. The use according to item 3, wherein the enzyme is ferulic acid decarboxylase 1 (SD5) of SEQ ID NO 2 or a modified sequence having a percentage of sequence identity of at least 85% with SEQ ID NO 2 and capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.
7. The use according to item 6, wherein the enzyme is ferulic acid decarboxylase 1 (SD5) of SEQ ID NO 2, or a modified sequence thereof characterized by comprising at least one or more mutations with respect to SEQ ID NO 2, wherein the modified sequence is characterized by comprising the amino acid sequence represented by SEQ ID NO 2 with at least one substitution in the corresponding amino acid position of the modified amino acid sequence with respect to SEQ ID NO 2 selected from the list of:

| Amino acid of SEQ ID NO 2 | Position in SEQ ID NO 2 | Amino acid of the modified sequence |
|---|---|---|
| H | 145 | A |
| H | 145 | D |
| H | 145 | F |
| H | 145 | G |
| H | 145 | K |
| H | 145 | L |
| H | 145 | N |
| H | 145 | P |
| H | 145 | S |
| Y | 156 | C |
| Y | 156 | D |
| Y | 156 | F |
| Y | 156 | G |
| Y | 156 | H |
| Y | 156 | L |
| Y | 156 | P |
| Y | 156 | S |
| I | 173 | C |
| I | 173 | E |
| I | 173 | F |
| I | 173 | G |
| I | 173 | K |
| I | 173 | P |
| I | 173 | T |
| I | 173 | V |
| L | 187 | A |
| L | 187 | E |
| L | 187 | F |
| L | 187 | G |
| L | 187 | M |
| L | 187 | P |
| L | 187 | R |
| L | 187 | T |
| I | 189 | A |
| I | 189 | D |
| I | 189 | F |
| I | 189 | G |
| I | 189 | K |
| I | 189 | P |
| I | 189 | T |
| I | 189 | V |
| P | 191 | A |
| P | 191 | E |
| P | 191 | G |
| P | 191 | K |
| P | 191 | L |
| P | 191 | Q |
| P | 191 | S |
| P | 191 | Y |
| Q | 192 | A |
| Q | 192 | E |
| Q | 192 | G |
| Q | 192 | H |
| Q | 192 | L |
| Q | 192 | P |
| Q | 192 | S |
| Q | 192 | Y |
| S | 227 | A |
| S | 227 | E |
| S | 227 | F |
| S | 227 | G |
| S | 227 | K |
| S | 227 | P |
| S | 227 | T |
| S | 227 | V |
| P | 229 | A |
| P | 229 | E |
| P | 229 | G |
| P | 229 | K |
| P | 229 | S |
| P | 229 | T |
| P | 229 | V |
| P | 229 | Y |
| M | 286 | A |
| M | 286 | E |
| M | 286 | F |
| M | 286 | G |
| M | 286 | H |
| M | 286 | P |
| M | 286 | S |
| M | 286 | V |
| M | 286 | Y |
| H | 287 | A |
| H | 287 | E |
| H | 287 | G |
| H | 287 | M |
| H | 287 | P |
| H | 287 | R |
| H | 287 | T |
| H | 287 | Y |
| F | 291 | A |
| F | 291 | E |
| F | 291 | G |
| F | 291 | H |
| F | 291 | L |
| F | 291 | P |
| F | 291 | S |
| F | 291 | Y |
| C | 298 | A |
| C | 298 | E |
| C | 298 | G |
| C | 298 | K |
| C | 298 | M |
| C | 298 | N |
| C | 298 | P |
| C | 298 | S |
| C | 298 | Y |
| S | 317 | C |
| S | 317 | D |
| S | 317 | F |
| S | 317 | G |
| S | 317 | P |
| S | 317 | R |
| S | 317 | T |
| S | 317 | V |
| P | 319 | A |
| P | 319 | E |
| P | 319 | F |
| P | 319 | G |
| P | 319 | K |
| P | 319 | Q |
| P | 319 | S |
| P | 319 | V |
| D | 324 | A |
| D | 324 | E |
| D | 324 | G |
| D | 324 | K |
| D | 324 | N |
| D | 324 | P |
| D | 324 | S |
| D | 324 | V |
| D | 324 | Y |
| E | 325 | A |
| E | 325 | D |
| E | 325 | G |
| E | 325 | K |
| E | 325 | L |
| E | 325 | P |
| E | 325 | Q |
| E | 325 | S |
| E | 325 | Y |
| T | 326 | A |
| T | 326 | E |
| T | 326 | F |
| T | 326 | G |
| T | 326 | I |
| T | 326 | K |
| T | 326 | N |
| T | 326 | P |
| T | 326 | S |
| I | 330 | A |
| I | 330 | E |
| I | 330 | G |
| I | 330 | L |
| I | 330 | P |
| I | 330 | R |
| I | 330 | T |
| I | 330 | W |
| V | 334 | A |
| V | 334 | E |
| V | 334 | F |
| V | 334 | G |
| V | 334 | I |
| V | 334 | P |
| V | 334 | R |
| V | 334 | T |
| A | 360 | C |
| A | 360 | E |
| A | 360 | F |
| A | 360 | G |
| A | 360 | P |
| A | 360 | R |
| A | 360 | T |
| A | 360 | V |
| L | 361 | C |
| L | 361 | D |
| L | 361 | F |
| L | 361 | G |
| L | 361 | P |
| L | 361 | R |
| L | 361 | T |
| L | 361 | V |
| V | 395 | A |
| V | 395 | E |
| V | 395 | F |
| V | 395 | G |
| V | 395 | H |
| V | 395 | L |
| V | 395 | P |
| V | 395 | S |
| V | 395 | Y |
| F | 397 | A |
| F | 397 | D |
| F | 397 | G |
| F | 397 | L |
| F | 397 | N |
| F | 397 | P |
| F | 397 | R |
| F | 397 | S |
| F | 397 | Y |
| I | 398 | A |
| I | 398 | D |
| I | 398 | F |
| I | 398 | G |
| I | 398 | L |
| I | 398 | N |
| I | 398 | P |
| I | 398 | R |
| I | 398 | S |
| F | 440 | C |
| F | 440 | D |
| F | 440 | G |
| F | 440 | I |
| F | 440 | P |
| F | 440 | R |
| F | 440 | T |
| F | 440 | Y |
| P | 441 | A |
| P | 441 | D |
| P | 441 | G |
| P | 441 | Q |
| P | 441 | R |
| P | 441 | S |
| P | 441 | V |
| P | 441 | Y |
| L | 442 | A |
| L | 442 | D |
| L | 442 | F |
| L | 442 | G |
| L | 442 | I |
| L | 442 | P |
| L | 442 | R |
| L | 442 | T |
| A | 443 | C |
| A | 443 | D |
| A | 443 | F |
| A | 443 | G |
| A | 443 | I |
| A | 443 | P |
| A | 443 | R |
| A | 443 | T |
| A | 443 | V |
| S | 447 | A |
| S | 447 | D |
| S | 447 | G |
| S | 447 | K |
| S | 447 | L |
| S | 447 | P |
| S | 447 | T |
| S | 447 | Y |

8. The use according to item 6, wherein the enzyme is ferulic acid decarboxylase 1 (SD5) of SEQ ID NO 2, or a modified sequence thereof characterized by comprising any of the double mutations with respect to SEQ ID NO 2, wherein the modified sequence is characterized by comprising the amino acid sequence represented by SEQ ID NO 2 with at least one double substitution in the corresponding amino acid positions of the modified amino acid sequence with respect to SEQ ID NO 2 selected from the list of:

| Position in SEQ ID NO 2 | Amino acid of the modified sequence | Position in SEQ ID NO 2 | Amino acid of the modified sequence |
|---|---|---|---|
| 145 | N | 286 | F |
| 145 | N | 317 | L |
| 145 | W | 286 | H |
| 145 | W | 317 | T |
| 156 | A | 326 | H |
| 156 | H | 326 | H |
| 156 | L | 319 | V |
| 156 | W | 319 | T |
| 156 | W | 326 | P |
| 187 | H | 286 | H |
| 187 | H | 291 | S |
| 187 | H | 298 | Y |
| 187 | P | 298 | H |
| 187 | P | 298 | Q |
| 187 | P | 298 | R |
| 187 | Q | 286 | F |
| 187 | R | 286 | H |
| 187 | R | 291 | D |
| 187 | R | 291 | N |
| 187 | R | 298 | F |
| 187 | R | 298 | Q |
| 189 | H | 291 | S |
| 189 | Q | 291 | S |
| 189 | Q | 291 | T |
| 191 | G | 298 | Q |
| 191 | G | 397 | A |
| 191 | H | 298 | Y |
| 191 | H | 397 | M |
| 229 | K | 330 | K |
| 229 | S | 330 | M |
| 229 | S | 398 | S |
| 286 | H | 298 | F |
| 286 | H | 317 | T |
| 287 | L | 442 | T |
| 287 | R | 447 | R |
| 287 | T | 442 | Y |
| 287 | Y | 447 | R |
| 319 | G | 326 | H |
| 319 | S | 324 | S |
| 319 | T | 326 | P |
| 319 | V | 324 | M |
| 319 | V | 324 | Q |
| 324 | N | 326 | G |
| 330 | M | 398 | S |
| 330 | M | 398 | Y |
| 330 | V | 398 | R |
| 334 | R | 361 | R |
| 334 | R | 361 | W |
| 334 | T | 361 | R |
| 442 | T | 447 | G |

9. A composition comprising a modified sequence having a percentage of sequence identity of at least 85% with any of sequences SEQ ID NO 1 to 6.
10. The composition of item 9, wherein the modified sequence has a percentage of sequence identity of at least 85% with SEQ ID NO 1 and is capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.
11. The composition of item 9, wherein the modified sequence of a decarboxylase enzyme is as defined in item 5.
12. The composition of item 9, wherein the modified sequence has a percentage of sequence identity of at least 85% with SEQ ID NO 2 and is capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.
13. The composition of item 9, wherein the modified sequence of a decarboxylase enzyme is as defined in item 7.
14. The composition of item 9, wherein the modified sequence of a decarboxylase enzyme is as defined in item 8.
15. A composition as defined in any one of items 9 to 14, for use in therapy.
16. A composition comprising an enzyme selected from the list consisting of cis-aconitate decarboxylase (SD1) of SEQ ID NO 1, ferulic acid decarboxylase 1 (SD5) of SEQ ID NO 2, or any of sequences SEQ ID NO 3 (SD2), SEQ ID NO 4 (SD3), SEQ ID NO 5 (SD4) or SEQ ID NO 6 (SD6), or a modified sequence having a percentage of sequence identity of at least 85% with any of sequences SEQ ID NO 1 to 6 and capable of reducing the circulating succinate levels in a patient in need thereof, for use in a method for the prophylactic and/or therapeutic treatment of a disease associated with increased levels of succinate in a patient.
17. The composition for use according to item 16, wherein the enzyme is cis-aconitate decarboxylase (SD1) of SEQ ID NO 1, or a modified sequence having a percentage of sequence identity of at least 85% with SEQ ID NO 1 and capable of reducing the circulating succinate levels in a patient in need thereof.
18. The composition for use according to item 16, wherein the enzyme is cis-aconitate decarboxylase (SD1) of SEQ ID NO 1, or a modified sequence of a decarboxylase enzyme as defined in item 5 and capable of reducing the circulating succinate levels in a patient in need thereof.
19. The composition for use according to item 16, wherein the enzyme is ferulic acid decarboxylase 1 (SD5) of SEQ ID NO 2 or a modified sequence having a percentage of sequence identity of at least 85% with SEQ ID NO 2 and capable of reducing the circulating succinate levels in a patient in need thereof.
20. The composition for use according to item 16, wherein the enzyme is ferulic acid decarboxylase 1 (SD5) of SEQ ID NO 2, or a modified sequence of a decarboxylase enzyme as defined in item 7 and capable of reducing the circulating succinate levels in a patient in need thereof.
21. The composition for use according to item 16, wherein the enzyme is ferulic acid decarboxylase 1 (SD5) of SEQ ID NO 2, or a modified sequence of a decarboxylase enzyme as defined in item 8 and capable of reducing the circulating succinate levels in a patient in need thereof.
22. The composition comprising an enzyme as defined in any of items 1 to 8, for use in a method of therapy for reducing the intestinal succinate.
23. The composition for use according to item 16, wherein the disease is selected from the list consisting of hypertension, ischemia, obesity, type 2 diabetes, cancer, non-alcoholic fatty liver disease (NAFLD), non-alcoholic steatohepatitis (NASH), inflammatory bowel disease (IBD), wherein IBD includes ulcerative colitis and Crohn's disease.
24. The composition for use according to item 23, wherein the disease is selected from the list consisting of obesity, type 2 diabetes mellitus, non-alcoholic fatty liver disease (NAFLD) and Crohn's disease.

## Claims

1. A composition comprising an enzyme selected from the list consisting of cis-aconitate decarboxylase (SD1) of SEQ ID NO 1, ferulic acid decarboxylase 1 (SD5) of SEQ ID NO 2, or any of sequences SEQ ID NO 3 (SD2), SEQ ID NO 4 (SD3), SEQ ID NO 5 (SD4) or SEQ ID NO 6 (SD6), or a modified sequence having a percentage of sequence identity of at least 85% with any of sequences SEQ ID NO 1 to 6, for use in therapy.

2. A composition comprising an enzyme selected from the list consisting of cis-aconitate decarboxylase (SD1) of SEQ ID NO 1 and ferulic acid decarboxylase 1 (SD5) of SEQ ID NO 2, or a modified sequence having a percentage of sequence identity of at least 85% with any of sequences SEQ ID NO 1 or 2, for use in a method for the prophylactic and/or therapeutic treatment of a disease associated with increased levels of succinate in a patient in need thereof.

3. The composition for use according to claim 2, wherein the enzyme is cis-aconitate decarboxylase (SD1) of SEQ ID NO 1, or a modified sequence having a percentage of sequence identity of at least 85% with SEQ ID NO 1.

4. The composition for use according to claim 3, wherein the enzyme is cis-aconitate decarboxylase (SD1) of SEQ ID NO 1, or a modified sequence thereof **characterized by** comprising at least one or more mutations with respect to SEQ ID NO 1, wherein the modified sequence is **characterized by** comprising the amino acid sequence represented by SEQ ID NO 1 with at least one substitution in the corresponding amino acid position of the modified amino acid sequence with respect to SEQ ID NO 1 selected from the list consisting of :
| Amino acid of SEQ ID NO 1 | Position in SEQ ID NO 1 | Amino acid of the modified sequence |
|---|---|---|
| L | 110 | W |
| H | 168 | A |
| H | 168 | K |
| K | 217 | A |
| K | 217 | Q |
| K | 288 | A |
| K | 288 | Q |
| G | 294 | A |
| H | 336 | A |
| H | 336 | K |

5. The composition for use according to claim 2, wherein the enzyme is ferulic acid decarboxylase 1 (SD5) of SEQ ID NO 2 or a modified sequence having a percentage of sequence identity of at least 85% with SEQ ID NO 2.

6. The composition for use according to claim 5, wherein the enzyme is ferulic acid decarboxylase 1 (SD5) of SEQ ID NO 2, or a modified sequence thereof **characterized by** comprising at least one or more mutations with respect to SEQ ID NO 2, wherein the modified sequence is **characterized by** comprising the amino acid sequence represented by SEQ ID NO 2 with at least one substitution in the corresponding amino acid position of the modified amino acid sequence with respect to SEQ ID NO 2 selected from the list consisting of:
| Amino acid of SEQ ID NO 2 | Position in SEQ ID NO 2 | Amino acid of the modified sequence |
|---|---|---|
| H | 145 | A |
| H | 145 | D |
| H | 145 | F |
| H | 145 | G |
| H | 145 | K |
| H | 145 | L |
| H | 145 | N |
| H | 145 | P |
| H | 145 | S |
| Y | 156 | C |
| Y | 156 | D |
| Y | 156 | F |
| Y | 156 | G |
| Y | 156 | H |
| Y | 156 | L |
| Y | 156 | P |
| Y | 156 | S |
| I | 173 | C |
| I | 173 | E |
| I | 173 | F |
| I | 173 | G |
| I | 173 | K |
| I | 173 | P |
| I | 173 | T |
| I | 173 | V |
| L | 187 | A |
| L | 187 | E |
| L | 187 | F |
| L | 187 | G |
| L | 187 | M |
| L | 187 | P |
| L | 187 | R |
| L | 187 | T |
| I | 189 | A |
| I | 189 | D |
| I | 189 | F |
| I | 189 | G |
| I | 189 | K |
| I | 189 | P |
| I | 189 | T |
| I | 189 | V |
| P | 191 | A |
| P | 191 | E |
| P | 191 | G |
| P | 191 | K |
| P | 191 | L |
| P | 191 | Q |
| P | 191 | S |
| P | 191 | Y |
| Q | 192 | A |
| Q | 192 | E |
| Q | 192 | G |
| Q | 192 | H |
| Q | 192 | L |
| Q | 192 | P |
| Q | 192 | S |
| Q | 192 | Y |
| S | 227 | A |
| S | 227 | E |
| S | 227 | F |
| S | 227 | G |
| S | 227 | K |
| S | 227 | P |
| S | 227 | T |
| S | 227 | V |
| P | 229 | A |
| P | 229 | E |
| P | 229 | G |
| P | 229 | K |
| P | 229 | S |
| P | 229 | T |
| P | 229 | V |
| P | 229 | Y |
| M | 286 | A |
| M | 286 | E |
| M | 286 | F |
| M | 286 | G |
| M | 286 | H |
| M | 286 | P |
| M | 286 | S |
| M | 286 | V |
| M | 286 | Y |
| H | 287 | A |
| H | 287 | E |
| H | 287 | G |
| H | 287 | M |
| H | 287 | P |
| H | 287 | R |
| H | 287 | T |
| H | 287 | Y |
| F | 291 | A |
| F | 291 | E |
| F | 291 | G |
| F | 291 | H |
| F | 291 | L |
| F | 291 | P |
| F | 291 | S |
| F | 291 | Y |
| C | 298 | A |
| C | 298 | E |
| C | 298 | G |
| C | 298 | K |
| C | 298 | M |
| C | 298 | N |
| C | 298 | P |
| C | 298 | S |
| C | 298 | Y |
| S | 317 | C |
| S | 317 | D |
| S | 317 | F |
| S | 317 | G |
| S | 317 | P |
| S | 317 | R |
| S | 317 | T |
| S | 317 | V |
| P | 319 | A |
| P | 319 | E |
| P | 319 | F |
| P | 319 | G |
| P | 319 | K |
| P | 319 | Q |
| P | 319 | S |
| P | 319 | V |
| D | 324 | A |
| D | 324 | E |
| D | 324 | G |
| D | 324 | K |
| D | 324 | N |
| D | 324 | P |
| D | 324 | S |
| D | 324 | V |
| D | 324 | Y |
| E | 325 | A |
| E | 325 | D |
| E | 325 | G |
| E | 325 | K |
| E | 325 | L |
| E | 325 | P |
| E | 325 | Q |
| E | 325 | S |
| E | 325 | Y |
| T | 326 | A |
| T | 326 | E |
| T | 326 | F |
| T | 326 | G |
| T | 326 | I |
| T | 326 | K |
| T | 326 | N |
| T | 326 | P |
| T | 326 | S |
| I | 330 | A |
| I | 330 | E |
| I | 330 | G |
| I | 330 | L |
| I | 330 | P |
| I | 330 | R |
| I | 330 | T |
| I | 330 | W |
| V | 334 | A |
| V | 334 | E |
| V | 334 | F |
| V | 334 | G |
| V | 334 | I |
| V | 334 | P |
| V | 334 | R |
| V | 334 | T |
| A | 360 | C |
| A | 360 | E |
| A | 360 | F |
| A | 360 | G |
| A | 360 | P |
| A | 360 | R |
| A | 360 | T |
| A | 360 | V |
| L | 361 | C |
| L | 361 | D |
| L | 361 | F |
| L | 361 | G |
| L | 361 | P |
| L | 361 | R |
| L | 361 | T |
| L | 361 | V |
| V | 395 | A |
| V | 395 | E |
| V | 395 | F |
| V | 395 | G |
| V | 395 | H |
| V | 395 | L |
| V | 395 | P |
| V | 395 | S |
| V | 395 | Y |
| F | 397 | A |
| F | 397 | D |
| F | 397 | G |
| F | 397 | L |
| F | 397 | N |
| F | 397 | P |
| F | 397 | R |
| F | 397 | S |
| F | 397 | Y |
| I | 398 | A |
| I | 398 | D |
| I | 398 | F |
| I | 398 | G |
| I | 398 | L |
| I | 398 | N |
| I | 398 | P |
| I | 398 | R |
| I | 398 | S |
| F | 440 | C |
| F | 440 | D |
| F | 440 | G |
| F | 440 | I |
| F | 440 | P |
| F | 440 | R |
| F | 440 | T |
| F | 440 | Y |
| P | 441 | A |
| P | 441 | D |
| P | 441 | G |
| P | 441 | Q |
| P | 441 | R |
| P | 441 | S |
| P | 441 | V |
| P | 441 | Y |
| L | 442 | A |
| L | 442 | D |
| L | 442 | F |
| L | 442 | G |
| L | 442 | I |
| L | 442 | P |
| L | 442 | R |
| L | 442 | T |
| A | 443 | C |
| A | 443 | D |
| A | 443 | F |
| A | 443 | G |
| A | 443 | I |
| A | 443 | P |
| A | 443 | R |
| A | 443 | T |
| A | 443 | V |
| S | 447 | A |
| S | 447 | D |
| S | 447 | G |
| S | 447 | K |
| S | 447 | L |
| S | 447 | P |
| S | 447 | T |
| S | 447 | Y |

7. The composition for use according to claim 5, wherein the enzyme is ferulic acid decarboxylase 1 (SD5) of SEQ ID NO 2, or a modified sequence thereof **characterized by** comprising at least one double mutation with respect to SEQ ID NO 2, wherein the modified sequence is **characterized by** comprising the amino acid sequence represented by SEQ ID NO 2 with at least one double substitution in the corresponding amino acid positions of the modified amino acid sequence with respect to SEQ ID NO 2 selected from the list consisting of:
| Position in SEQ ID NO 2 | Amino acid of the modified sequence | Position in SEQ ID NO 2 | Amino acid of the modified sequence |
|---|---|---|---|
| 145 | N | 286 | F |
| 145 | N | 317 | L |
| 145 | W | 286 | H |
| 145 | W | 317 | T |
| 156 | A | 326 | H |
| 156 | H | 326 | H |
| 156 | L | 319 | V |
| 156 | W | 319 | T |
| 156 | W | 326 | P |
| 187 | H | 286 | H |
| 187 | H | 291 | S |
| 187 | H | 298 | Y |
| 187 | P | 298 | H |
| 187 | P | 298 | Q |
| 187 | P | 298 | R |
| 187 | Q | 286 | F |
| 187 | R | 286 | H |
| 187 | R | 291 | D |
| 187 | R | 291 | N |
| 187 | R | 298 | F |
| 187 | R | 298 | Q |
| 189 | H | 291 | S |
| 189 | Q | 291 | S |
| 189 | Q | 291 | T |
| 191 | G | 298 | Q |
| 191 | G | 397 | A |
| 191 | H | 298 | Y |
| 191 | H | 397 | M |
| 229 | K | 330 | K |
| 229 | S | 330 | M |
| 229 | S | 398 | S |
| 286 | H | 298 | F |
| 286 | H | 317 | T |
| 287 | L | 442 | T |
| 287 | R | 447 | R |
| 287 | T | 442 | Y |
| 287 | Y | 447 | R |
| 319 | G | 326 | H |
| 319 | S | 324 | S |
| 319 | T | 326 | P |
| 319 | V | 324 | M |
| 319 | V | 324 | Q |
| 324 | N | 326 | G |
| 330 | M | 398 | S |
| 330 | M | 398 | Y |
| 330 | V | 398 | R |
| 334 | R | 361 | R |
| 334 | R | 361 | W |
| 334 | T | 361 | R |
| 442 | T | 447 | G |

8. The composition for use according to any one of claims 2 to 7, wherein the disease is selected from the list consisting of hypertension, ischemia, obesity, type 2 diabetes, cancer, non-alcoholic fatty liver disease (NAFLD), non-alcoholic steatohepatitis (NASH), inflammatory bowel disease (IBD), wherein IBD includes ulcerative colitis and Crohn's disease.

9. The composition for use according to claim 8, wherein the disease is selected from the list consisting of obesity, inflammatory bowel disease (IBD), type 2 diabetes mellitus, non-alcoholic fatty liver disease (NAFLD) and Crohn's disease.

10. A composition comprising a modified sequence of a decarboxylase enzyme as defined in any one of claims 1 to 9.

11. The composition of claim 10, wherein the modified sequence of a decarboxylase enzyme has a percentage of sequence identity of at least 85% with SEQ ID NO 1 and is capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

12. The composition of claim 10, wherein the modified sequence of a decarboxylase enzyme is as defined in claim 4.

13. The composition of claim 10, wherein the modified sequence of a decarboxylase enzyme has a percentage of sequence identity of at least 85% with SEQ ID NO 2 and is capable of carrying out a biochemical enzymatic reaction involving said decarboxylase enzyme and succinate as the substrate of the enzyme to convert succinate to at least a product, thereby reducing the concentration of the substrate in comparison to the initial concentration of the substrate prior to carrying out the biochemical enzymatic reaction.

14. The composition of claim 10, wherein the modified sequence of the decarboxylase enzyme is as defined in claim 6.

15. The composition of claim 10, wherein the modified sequence of the decarboxylase enzyme is as defined in claim 7.
